# EUROPEAN PATENT SPECIFICATION

(11) **EP 2 282 208 B1**
(45) Date of publication and mention of the grant of the patent: **23.11.2011**
(21) Application number: 09167060.4
(22) Date of filing: 03.08.2009
(51) Int. Cl.: G01N 33/574, C12N 15/00, A61K 48/00

(54) **Methods and compounds for the diagnosis and treatment of cancer**
Verfahren und Verbindungen zur Diagnose und Behandlung von Krebs
Procédés et composés pour le diagnostic et le traitement du cancer

(43) Date of publication of application: 09.02.2011
(73) Proprietor: Universitätsklinikum Freiburg, 79106 Freiburg (DE)
(72) Inventor: Schüle, Roland, 79367 Weisweil (DE); Metzger, Eric, 68600 Neuf-Brisach (FR)
(74) Representative: Bühler, Dirk

(56) References cited:
- EP-A- 2 017 621
- COOPER C S ET AL: "Concepts of epigenetics in prostate cancer development" BRITISH JOURNAL OF CANCER, vol. 100, no. 2, January 2009 (2009-01), pages 240-245, XP002562022 ISSN: 0007-0920(print) 1532-1827(ele
- KIM JEEWON ET AL: "Centrosomal PKC beta II and pericentrin are critical for human prostate cancer growth and angiogenesis" CANCER RESEARCH, vol. 68, no. 16, August 2008 (2008-08), pages 6831-6839, XP009109086 ISSN: 0008-5472
- SLEDGE JR G W ET AL: "Protein Kinase C-[beta] as a Therapeutic Target in Breast Cancer" SEMINARS IN ONCOLOGY 200606 US, vol. 33, no. SUPPL. 9, June 2006 (2006-06), pages 15-18, XP008116803 ISSN: 0093-7754
- MUSSELMAN C A ET AL: "Binding of the CHD4 PHD2 finger to histone H3 is modulated by covalent modifications" BIOCHEMICAL JOURNAL 20091015 PORTLAND PRESS LTD GBR, vol. 423, no. 2, 15 October 2009 (2009-10-15), pages 179-187, XP008116802

## Description

### FIELD OF THE INVENTION

The present invention provides in vitro methods for detecting, grading or prognosticating cancer, in particular prostate cancer.

### BACKGROUND OF THE INVENTION

Prostate cancer represents one of the most common types of cancer in men in the developed world and is the second leading cause of male cancer death. Prostate cancer is most often discovered by prostate-specific antigen (PSA) screening and less commonly by physical examination or by symptoms. However, there is some current concern about the accuracy of the PSA test and its usefulness, as said test has been found to often result in false positive or negative results.

Furthermore, the test does not allow the differentiation of benign prostate hyperplasia, non-aggressive prostate cancer and aggressive prostate cancer.

Therefore novel prostate cancer biomarkers are needed in order to improve the early detection and accuracy of diagnosis, to determine the aggressiveness of prostate cancer and to monitor the efficacy of treatment.

The growth and development of prostate cancer is stimulated by androgens (such as e.g. testosterone) and androgen receptor-regulated genes are pivotal to the control of tumour cell proliferation in prostate cancer. Antiandrogens can block the action of androgens and are therefore often employed in hormone therapy during treatment of prostate cancer. Other common treatment options include surgery and radiation. However, all of these treatment methods are associated with a high risk of side effects, including e.g. loss of bladder control, rectal problems, impotence and, in the case of hormone therapy, increased risk of heart attack, weight gain and liver damage. In addition, many patients ultimately become unresponsive to hormonal medications. The development of alternative pharmacological treatments for prostate cancer therefore remains an essential task.

Protein kinase C (PKC) comprises a superfamily of at least 12 isozymes that are activated in response to various stimuli. PKC isozymes are classified as conventional (α, β1, β2, γ), novel (δ, ε, η, θ, µ), and atypical (ζ, λ) isozymes. Conventional PKC isozymes are Ca²+-dependent, while novel and atypical isozymes do not require Ca²⁺ for their activation. All PKC isozymes, with the exception of ζ, λ, are activated by diacylglycerol (DAG).

PKC isozymes are serine/threonine kinases that act by catalyzing the transfer of a phosphate group from ATP to serine and threonine residues of their substrate proteins. They phosphorylate a wide variety of intracellular target proteins and have multiple functions in signal transduction-mediated cellular regulation. Activation of one or more PKC isoforms leads to a variety of biological responses, including changes in cell proliferation and differentiation, transmembrane ion transport, glucose and lipid metabolism, smooth muscle contraction, and gene expression. PKC isozymes negatively or positively regulate critical cell cycle transitions, including cell cycle entry and exit and the G₁ and G₂ checkpoints.

Thus, PKCs represent promising targets for the treatment of cancer.

However, given the complexity of PKC signaling, general inhibition of all PKC isoforms would result in severe side effects that might even endanger the survival of a patient. Therefore there is an ongoing need for isoform specific therapeutic PKC inhibitors.

The review article by Cooper and Foster entitled "Concepts of epigenetics in prostate cancer development", British Journal of Cancer, Vol. 100, No. 2, January 2009, pages 240-245 discloses *inter alia* histone-patterns which appear to be linked to prostate cancer and prostate cancer prognosis. Further biomarkers such as EZH2, BMII and RING1 are also described therein.

The article by Kim j et al., Cancer Research, Vol. 68, No. 16, August 2008, pages 6831-6839 entitled "Centrosomal PKCβII and Pericentrin are critical for human prostate cancer growth and angiogenesis" suggests that PKCβII inhibitors may be used to reduce prostate cancer growth by targeting both angiogenesis and tumor cell growth.

The review article by Sledge and Gökmen-Polar entitled "protein kinase C-β as a therapeutic target in breast cancer", Seminars in oncology, Vol. 33, No. suppl. 9, June 2006, pages 15-18 focuses on the rationale for using PKC-β as a therapeutic target at both the preclinical and clinical levels in breast cancer.

EP 2 017 621 in the name of the Universitätsklinikum Freiburg (published on January 21, 2009) discloses a novel epigenetic mark for transcriptional regulation, namely histone H3 phosphorylated at threonine 11. Furthermore, a process for controlling at least one androgen receptor- (AR-) regulated mechanism in mammalian cells under histone-phosphorylating conditions is disclosed therein.

### Objective and summary of the invention

It is one object of the present invention to provide methods that can be used for detecting, grading and/or prognosticating cancer, in particular prostate cancer, in a sample from a subject.

This and other objectives as they will become apparent from the ensuing description and claims are attained by the subject matter of the independent claims. Some of the preferred embodiments are defined by the dependent claims.

In a first aspect the present invention provides a method for detecting, grading and/or prognosticating cancer comprising the step of determining in a sample from a subject the amount of histone H3 phosphorylated at threonine 6 (H3T6ph).

In another aspect the present invention further provides a diagnostic kit for detecting, grading, and/or prognosticating cancer comprising a detecting agent specific for histone H3 phosphorylated at threonine 6 (H3T6ph) , wherein the detecting agent is an antibody, an aptamer or an oligonucleotide probe.

In yet another aspect the present invention relates to the use of a detecting agent specific for histone H3 phosphorylated at threonine 6 (H3T6ph) for detecting, grading and/or prognosticating cancer in a sample from a subject, wherein the detecting agent is an antibody, an aptamer or an oligonucleotide probe.

### BRIEF DESCRIPTION OF THE FIGURES

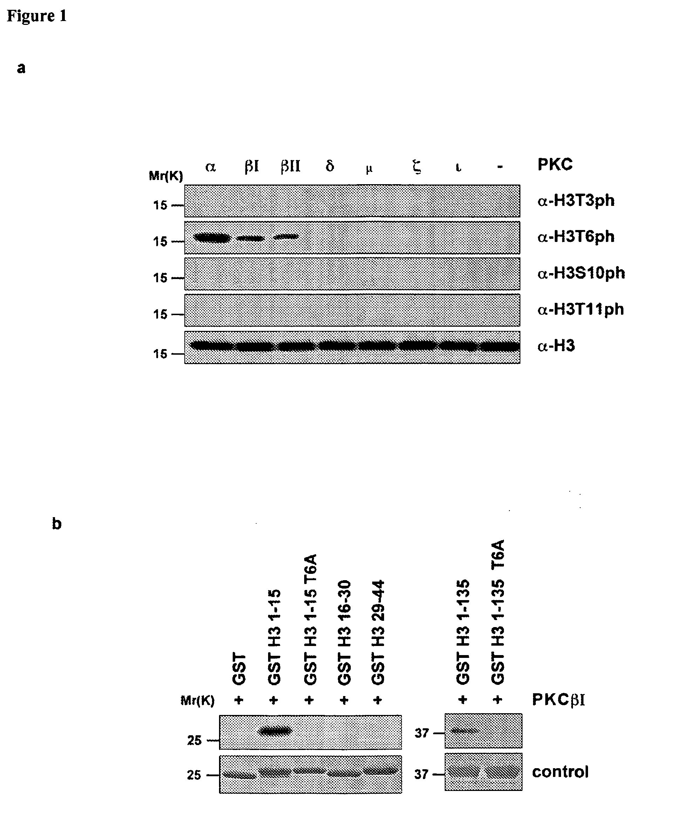

**Figure 1** PKCα, βI and βII specifically phosphorylate histone H3 at threonine 6 (H3T6). Nucleosomes from HeLa cells (**a**) or bacterially expressed GST and GST-H3 proteins (**b**) were incubated with active PKCα, βI, βII,δ,µ,ζ or τ, as indicated. Coomassie blue staining (**b**, bottom panel) shows the amounts of GST fusion proteins used. Western blots were decorated with the indicated antibodies (**a**).

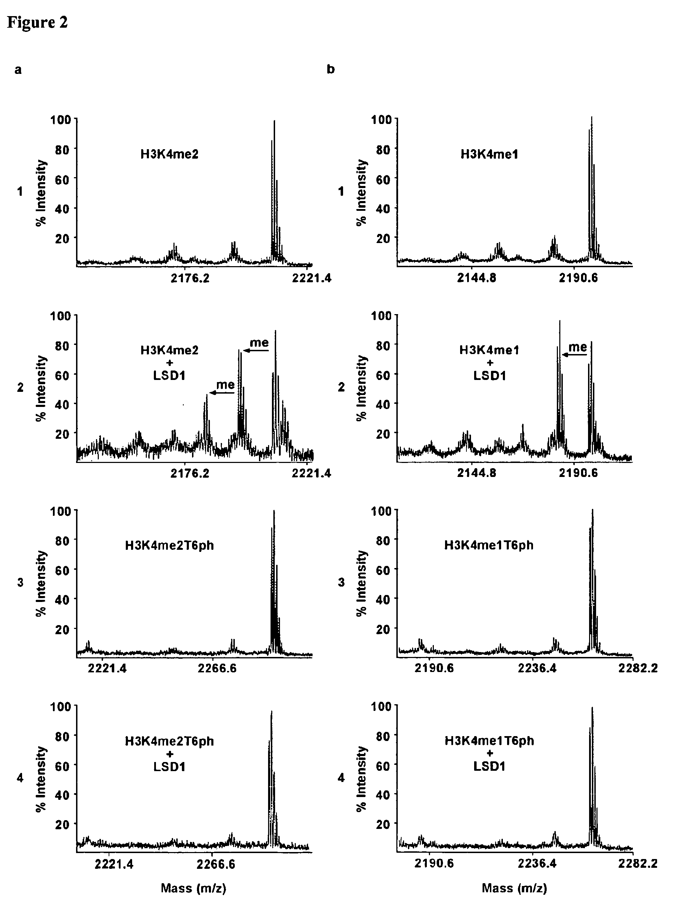

**Figure 2** Phosphorylation of H3T6 blocks demethylation at lysine 4 of histone H3 (H3K4) by LSD1. H3K4me2 or H3K4me2T6ph (**a**) and H3K4me1 or H3K4me1 phosphorylated at threonine 6 (H3K4me1T6ph) (**b**) peptides corresponding to the H3 tail residues 1-20 were incubated in the presence (**a**, **b**; panel 2 and 4) or in the absence of LSD1 (**a**, **b**; panel 1 and 3) and analysed by mass spectrometry. A shift in mass equivalent to one methyl group is indicated as "me".

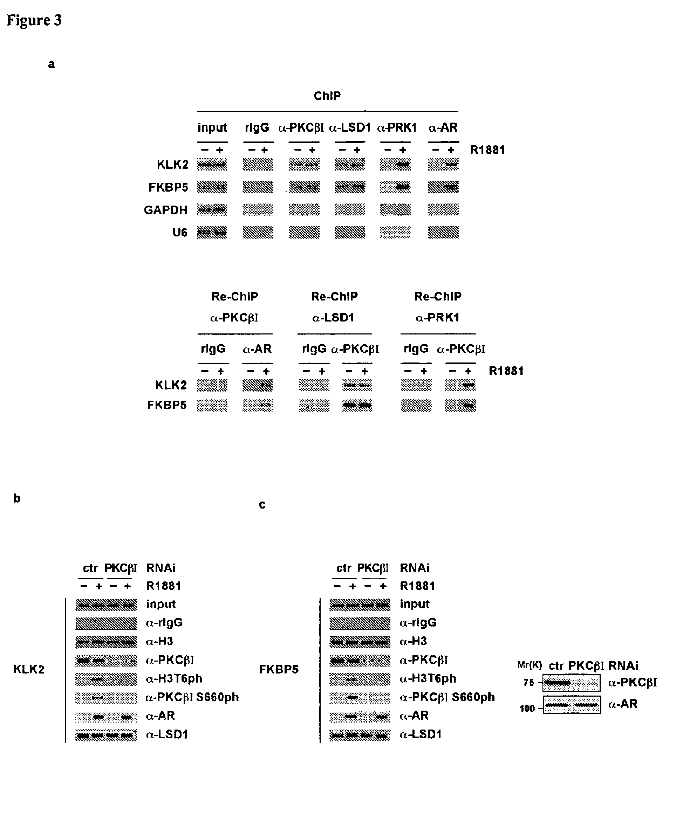

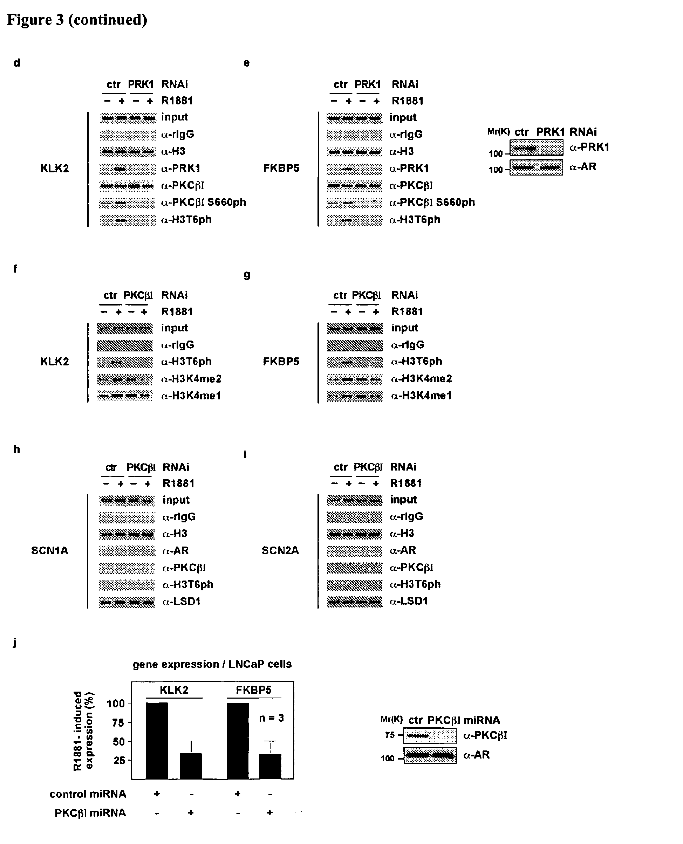

**Figure 3** PKCβI phosphorylates H3T6 and controls demethylation of H3K4 during AR-dependent gene expression. For ChIP (**a-i**), *LNCaP cells were cultivated in the presence or absence of the AR agonist R1881 and transfected with siRNA (**b-i**). ChIP analyses were performed with the indicated antibodies. The precipitated chromatin was amplified by PCR using primers flanking androgen response elements (AREs) in the promoter region of the *KLK2* and *FKBP5* genes or primers in the promoter region of the *GAPDH, U6, SCN1A,* and *SCN2A* genes. (**j**), miRNA-mediated PKCβI knockdown reduces expression of the androgen-regulated *KLK2* and *FKBP5* genes in LNCaP cells. Bars represent mean +SD (n≥3). Western blots (**c**, **e**, j, right panels) were decorated with the indicated antibodies.

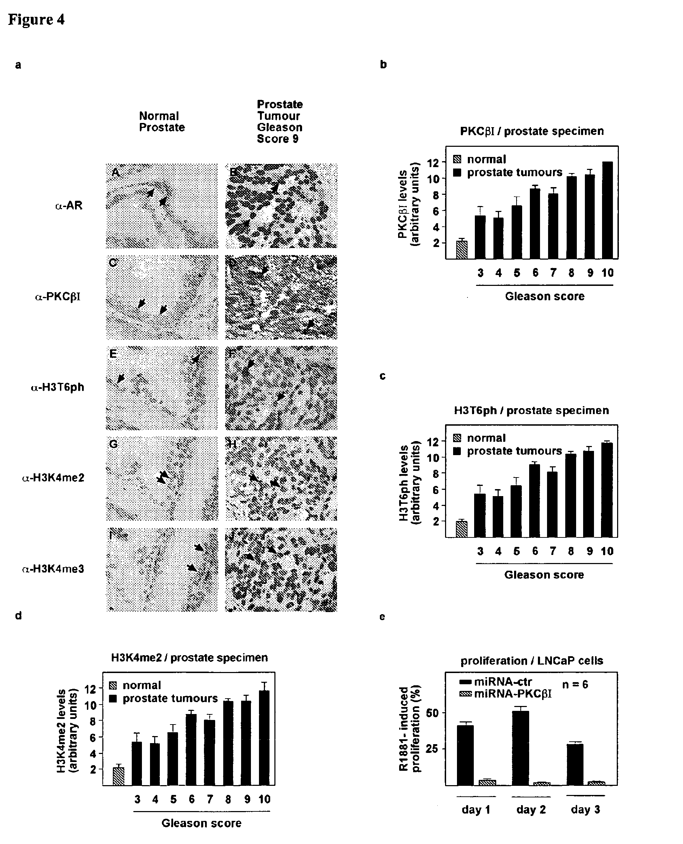

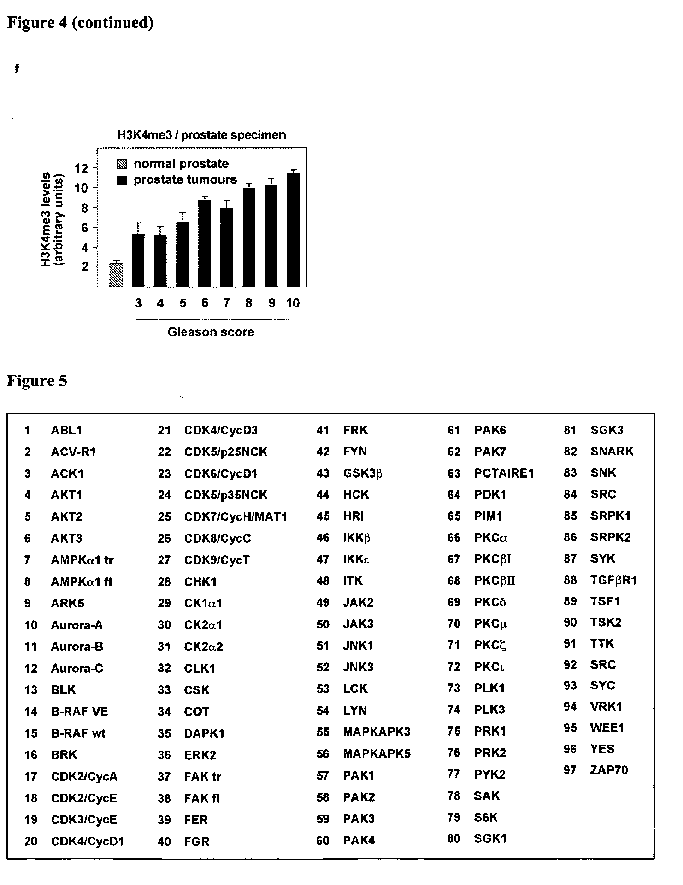

**Figure 4** PKCβI, H3T6ph, H3K4me2 and H3K4me3 levels positively correlate with the malignancy of prostate cancer and control androgen-dependent tumour cell proliferation. (**a**), immunohistochemical staining of AR, PKCβI, H3T6ph, H3K4me2 and H3K4me3 in human normal and tumour prostate. AR (A, B), PKCβI (C, D), H3T6ph (E, F), H3K4me2 (G, H), and H3K4me3 (I, J) immunoreactivity is detected in the secretory epithelium of normal prostate (A, C, E, G, I, arrows) and prostate carcinoma cells (B, D, F, H, J, arrows). All sections were taken from the same radical prostatectomy specimen (magnification: x250). The correlation of elevated PKCβI, H3T6ph, H3K4me2 and H3K4me3 levels with high Gleason score in a panel of 154 human prostate carcinomas is highly significant: r=0.5292, p<0.0001 (**b**); r=0.5386, p<0.0001 (**c**); r=0.5373, p<0.0001 (**d**); r=0.5395, p<0.0001 (**f**). Normal prostate specimens are included as a control. (**e**), miRNA-mediated PKCβI knockdown severely reduces R1881-induced cell proliferation in LNCaP cells. Bars represent mean +SD (n≥6).
**Figure 5** Alphabetical list of the 97 kinases (ProQinase GmbH) used to screen for H3T6 specific kinases.

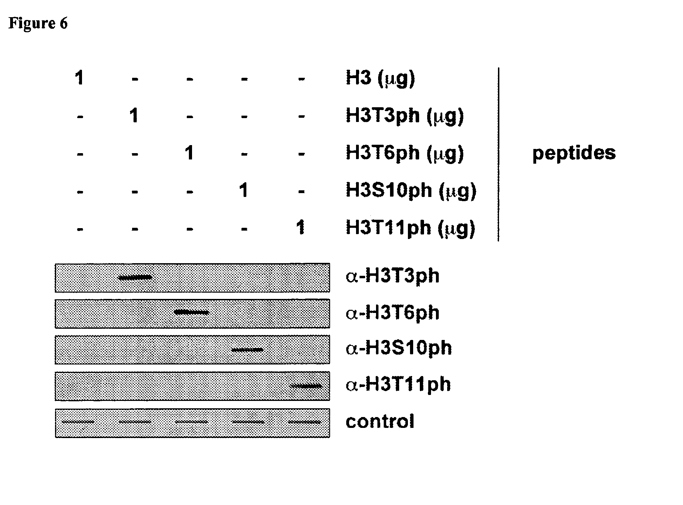

**Figure 6** The α-H3T3ph²¹, α-H3T6ph (# ab14102 lot 481643; Abcam), α-H3S10ph (# 06-570 lot 32219; Upstate), and α-H3T11ph³ antibodies used for Western blot analysis or ChIP assays specifically recognize histone H3 phosphorylated at threonine 3 (H3T3ph), histone H3 phosphorylated at threonine 6 (H3T6ph), histone H3 phosphorylated at serine 10 (H3S10ph), and histone H3 phosphorylated at threonine 11 (H3T11ph), respectively. The indicated amounts of peptides were spotted onto nitrocellulose (Protran BA 79, Schleicher & Schuell). H3T3ph, H3T6ph, H3S10ph, and H3T11ph peptides were obtained from Abcam. The unmodified H3 peptide was obtained from Peptides & Elephants. Western blots were decorated as indicated. Control shows the amounts of Ponceau red stained peptides (bottom panel).

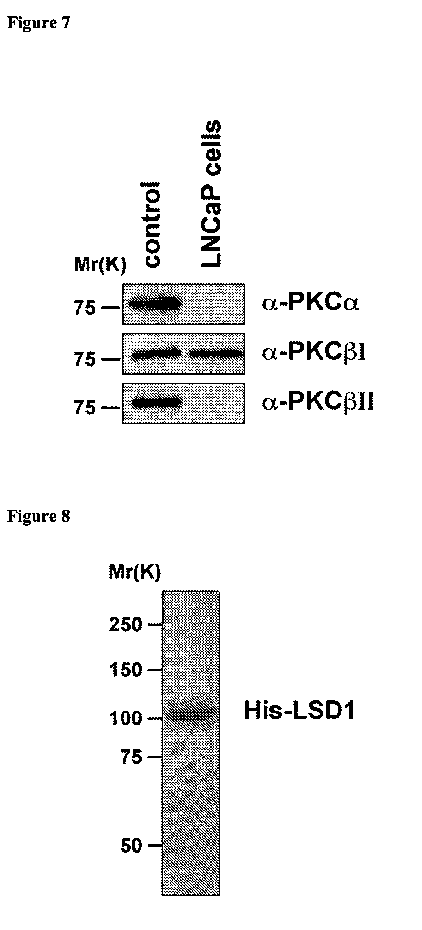

**Figure 7** PKCβI but not PKCα or PKCβII is expressed in LNCaP cells. Western blots were decorated with the indicated antibodies.
**Figure 8** Coomassie blue staining shows the bacterially expressed and purified His-LSD1 protein used in the demethylation assay.

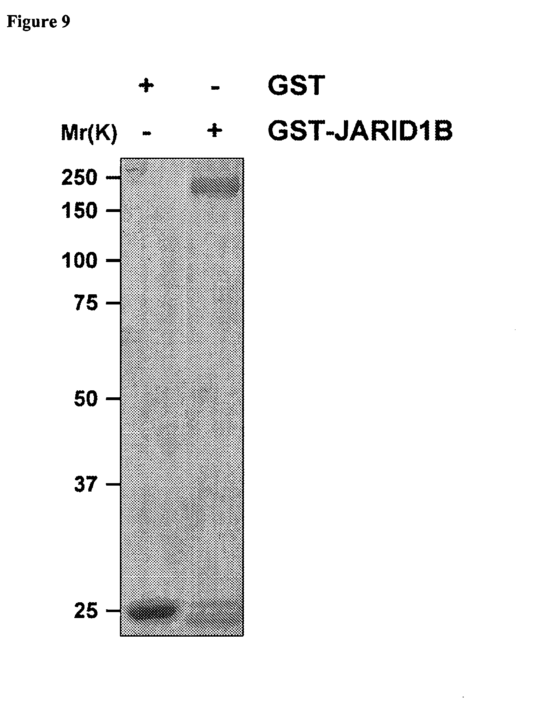

**Figure 9** Coomassie blue staining shows the purified GST and GST-JARID1B protein used in the demethylation assay.

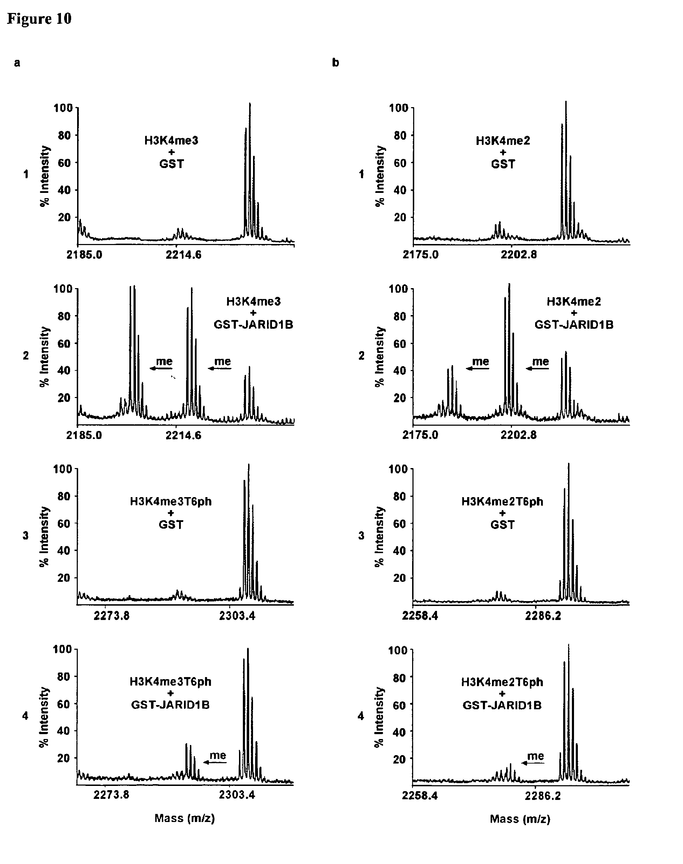

**Figure 10** Phosphorylation of H3T6 blocks demethylation at H3K4 by JARID1B. H3K4me3 or H3K4me3T6ph (**a**) and H3K4me2 or H3K4me2T6ph (**b**) peptides corresponding to the H3 tail residues 1-20 were incubated with GST (**a**, **b**; panel 1 and 3) or GST-JARID1B (**a**, **b**; panel 2 and 4) and analysed by mass spectrometry. A shift in mass equivalent to one methyl group is indicated as "me".

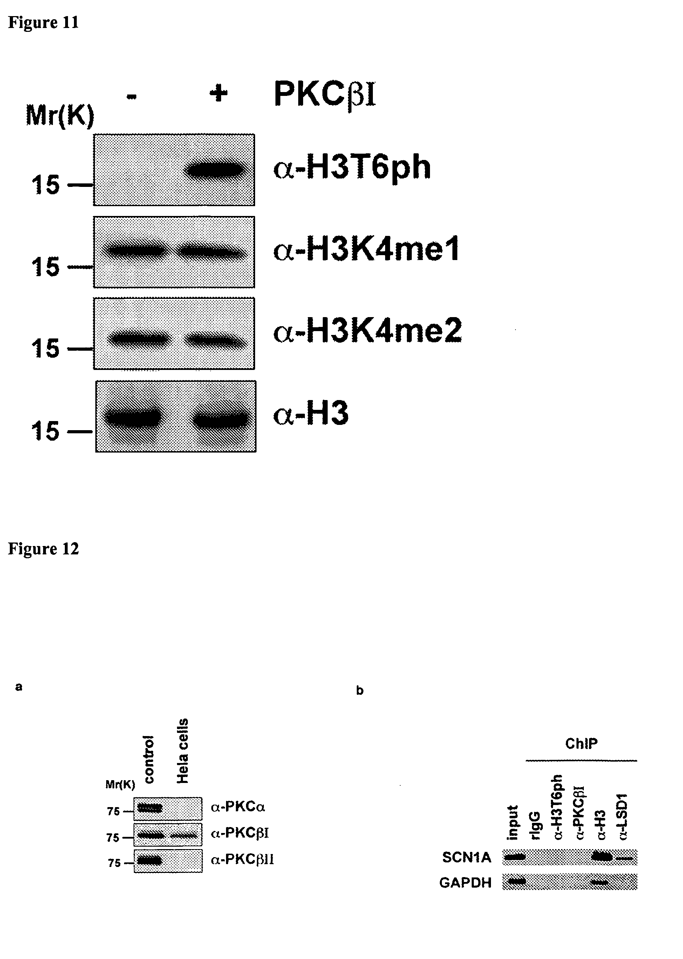

**Figure 11** The specificity of the α-H3K4me2 and α-H3K4me1 antibodies is not altered by phosphorylation of H3T6. Equal amounts of either untreated nucleosomes (-) or nucleosomes phosphorylated at H3T6 *in vitro* by PKC (+) were used for Western blot analysis. Western blots were decorated with the indicated antibodies.
**Figure 12** PKCβI is expressed in HeLa cells (**a**), but does not interact with the chromatinized *SCN1A* promoter (**b**). ChIP analyses were performed with the indicated antibodies. The precipitated chromatin was amplified by PCR using primers in the promoter region of *SCN1A* and *GAPDH.* Western blots (**a**) were decorated with the indicated antibodies.

### DETAILED DESCRIPTION OF THE INVENTION

Unless defined otherwise, all technical and scientific terms used herein have the same meanings as commonly understood by one of ordinary skill in the art.

The following definitions are introduced:
It is to be understood that the term "comprise", and variations such as "comprises" and "comprising" is not limiting. For the purpose of the present invention the term "consisting of" is considered to be a preferred embodiment of the term "comprising". If hereinafter a group is defined to comprise at least a certain number of embodiments, this is meant to also encompass a group which preferably consists of these embodiments only.

The terms "about" and "approximately" in the context of the present invention denote an interval of accuracy that a person skilled in the art will understand to still ensure the technical effect of the feature in question. The term typically encompasses a deviation from the indicated numerical value of ±10 % and preferably of ±5 %.

The determination of percent identity between two sequences is preferably accomplished using the mathematical algorithm of Karlin and Altschul (1993) Proc. Natl. Acad. Sci USA 90: 5873-5877. Such an algorithm is e.g. incorporated into the BLASTn and BLASTp programs of Altschul et al. (1990) J. MoI. Biol. 215: 403-410 available at NCBI (http://www.ncbi.nlm.nih.gov/blast/Blast.cge).

The determination of percent identity is preferably performed with the standard parameters of the BLASTn and BLASTp programs.

BLAST polynucleotide searches are preferably performed with the BLASTn program.

For the general parameters, the "Max Target Sequences" box may be set to 100, the "Short queries" box may be ticked, the "Expect threshold" box may be set to 10 and the "Word Size" box may be set to 28. For the scoring parameters the "Match/mismatch Scores" may be set to 1,-2 and the "Gap Costs" box may be set to linear. For the Filters and Masking parameters, the "Low complexity regions" box may not be ticked, the "Species-specific repeats" box may not be ticked, the "Mask for lookup table only" box may be ticked, the "Mask lower case letters" box may not be ticked.

BLAST protein searches are preferably performed with the BLASTp program.

For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension:1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked.

One letter amino acid abbreviations used herein correspond to IUPAC nomenclature (see e.g. European Journal of Biochemistry, 138:9-37, 1984).

The term "RNA interference" or "RNAi" as used herein refers to an RNA induced block of gene expression in a specific and post-transcriptional manner by degradation of a specific target mRNA.

The term "Gleason score" as used herein refers to a means of grading prostate cancer. Cancers with a higher Gleason score are more aggressive and have a worse prognosis. The Gleason score may e.g. be determined based upon the microscopic appearance of a tumor sample. The Gleason score may further be determined by assigning a grade to the most common tumor pattern and a second grade to the next most common tumor pattern in a sample from the tumor and adding the two grades to get the Gleason score. The Gleason grade typically ranges from 1 to 5 with 5 having the worst prognosis, while the Gleason score typically ranges from 2 to 10 with 10 having the worst prognosis.

The terms "cancer" or "tumor" as used herein are meant to include different types of cancers such as e.g. prostate cancer, breast cancer, colorectal cancer, lung cancer, blood cancer and cancers of the brain. Other cancers or tumors may include e.g. non-Hodgkin lymphoma, head and neck cancer, non-small cell lung cancer, ovarian cancer or urinary bladder cancer. One preferred type of cancer in the context of the present invention is prostate cancer. Another preferred type of cancer in the context of the present invention is breast cancer.

The term "isolated" in the context of the present invention indicates that a polynucleotide has been removed from its natural environment and/or is presented in a form in which it is not found in nature.

The term "subject" as used herein preferably refers to a human. However, veterinary applications are also in the scope of the present invention.

The term "subject" can therefore also refer to an animal, preferably a mammal such as e.g. non-human primates, mice, rats, rabbits, guinea pigs, dogs, cats, cattle, horses, sheep, pigs, goats and the like.

The term "aptamer" as used herein refers to a polynucleotide that has a specific binding affinity for a target compound or molecule of interest, e.g. a protein. Aptamers may e.g. be RNA, single stranded DNA, modified RNA or modified DNA molecules. The preparation of aptamers is well known in the art and may involve, inter alia, the use of combinatorial RNA libraries to identify binding sites (reference may e.g. be made to Gold (1995), Ann. Rev. Biochem 64, 763-797).

Histones are the main protein components of chromatin. They form the unit around which DNA is coiled in the nucleosomes of eukaryotic chromosomes. Histone H3 is one of the five main histone proteins involved in the structure of chromatin in eukaryotic cells. Histones may be modified by posttranslational modifications such as e.g. lysine acetylation, lysine methylation, lysine ubiquitylation, arginine methylation or serine, threonine or tyrosine phosphorylation.

The terms "histone H3" or "H3" as used herein may refer to any known histone H3 or variants thereof. Preferably, said histone H3 is mammalian histone H3, most preferably human histone H3.

In some embodiments histone H3 may for example have an amino acid sequence selected from any of SEQ ID NO: 7, 8 or 9 (corresponding to accession number AAA63185.1, accession number NP_066403.2 and accession number CAA88778.1 respectively) or variants thereof. Preferably, said variants are naturally occuring variants. If histone H3 has an amino acid sequence selected from any of SEQ ID NO: 7, 8 or 9, histone H3 may lack the N-terminal methionine residue of said sequence (e.g. due to a post-translational loss of said residue).

Thus, in one preferred embodiment histone H3 has an amino acid sequence selected from any of SEQ ID NO: 7, 8 or 9, wherein said sequence lacks the N-terminal methionine residue.

In some preferred embodiments Histone H3 consists of an amino acid sequence that is at least 70%, 75%, 80%, 85%, 86%, 87%, 88%, 89%, 90%, 91%, 92%, 93%, 94%, 95%, 96%, 97%, 98% or 99% identical to the amino acid sequence according to any of SEQ ID NO: 7, 8 or 9.

The position of amino acid residues within the histone H3 sequence, e.g. threonine 6 (T6) or lysine 4 (K4), is indicated herein with respect to the position of residues in the sequence according to any of SEQ ID NO: 7, 8 or 9, starting from the N-terminal end and sequentially numbering the amino acid residues wherein the number '0' is assigned to the N-terminal methionine residue, which is typically removed from the mature H3 protein in vivo by post-translational modification. In some embodiments where histone H3 has an amino acid sequence different from SEQ ID NO: 7, 8 or 9, the amino acid residues corresponding to threonine 6 and/or lysine 4 (or any other residue of interest) of SEQ ID NO: 7, 8 or 9 may be shifted to another position within said amino acid sequence (e.g. due to amino acid insertions or deletions). In such a case, the amino acids corresponding to threonine 6 and/or lysine 4 (or any other residue of interest) of SEQ ID NO: 7, 8 or 9 may e.g. be determined by aligning the sequence of said histone H3 to any or all of SEQ ID NO: 7, 8 or 9 and detecting the amino acid residues in said sequence which align to threonine 6 and/or lysine 4 (or any other residue of interest) of SEQ ID NO: 7, 8 and/or 9. The skilled person knows how to perform such sequence alignments. For example, blast programs publicly available at NCBI may be used. Preferably, said programs are used with standard parameters.

In one embodiment, threonine 6 and lysine 4 of histone H3 as used in the methods of the invention are comprised in the following consensus sequence of histone H3:
ARXKXTAR;
wherein X may be any amino acid.

The inventors of the present invention have surprisingly found that protein kinase C ß1 (herein also referred to as PKCßI) phosphorylates histone H3 at threonine 6 and that said phosphorylation blocks demethylation of methylated lysine 4 of histone H3 during androgen receptor (also referred to herein as "AR") induced gene expression. The inventors of the present invention have also surprisingly found that elevated levels of protein kinase C ß1 (PKCßI), histone H3 phosphorylated at threonine 6 (herein also referred to as H3T6ph), histone H3 monomethylated at lysine 4 (herein also referred to as H3K4me1), histone H3 dimethylated at lysine 4 (herein also referred to as H3K4me2) and histone H3 trimethylated at lysine 4 (herein also referred to as H3K4me3) correlate with high Gleason scores in human prostate carcinoma. The present disclosure thus discloses protein kinase C βI (PKCßI), histone H3 phosphorylated at threonine 6 (H3T6ph), histone H3 monomethylated at lysine 4 (H3K4me1), histone H3 dimethylated at lysine 4 (H3K4me2) and histone H3 trimethylated at lysine 4 (H3K4me3) as novel diagnostic markers for cancer, in particular prostate cancer, which provide the advantage that they can also be used to determine the grade and thus the aggressiveness of a given cancer and to monitor the efficacy of treatment of cancer. Furthermore, several of all of the diagnostic biomarkers according to the disclosure may be analyzed in parallel within the same sample from a subject thus improving accuracy of detecting cancer in a sample from a subject.

The present invention in a first aspect relates to a method for detecting, grading and/or prognosticating cancer comprising the step of determining in a sample from a subject the amount of histone H3 phosphorylated at threonine 6 (H3T6ph).

The methods according to the invention are performed in vitro.

In one embodiment, determining the amount of H3T6ph may e.g. be achieved by contacting the sample from the subject with a detecting agent specific for H3T6ph, i.e. the amount of H3T6ph may be detected by contacting the sample from the subject with a detecting agent specific for H3T6ph. A detecting agent is specific for a given target, if it binds said target with a higher affinity than any other compound in a sample (i.e. a non-target). For example, an antibody is specific for H3T6ph if it binds to H3T6ph with an affinity higher than the affinity with which it binds to H3K4me1, H3K4me2, H3K4me3 or any other compound present in the sample. Preferably, a detecting agent specific for a given target binds to said target only and does not bind at all to a non-target.

A suitable detecting agent may preferably be an antibody or an aptamer. Preferably, the antibody is a monoclonal or polyclonal antibody. In some embodiments the detecting agent may also be selected from antibody variants or fragments such as e.g. single chain antibodies, diabodies, minibodies, single chain Fv fragments (sc(Fv)), sc(Fv)₂ antibodies, Fab fragments or a F(ab')₂ fragments. In one preferred embodiment commercially available antibodies specific for H3T6ph may be used. Examples of preferred commercially available antibodies are described herein below in the example section.

Antibodies may be produced according to any suitable method known to the person skilled in the art. Polyclonal antibodies may e.g. be produced by immunization of animals with the antigen of choice, whereas monoclonal antibodies of defined specificity may e.g. be produced using the hybridoma technology developed by Köhler and Milstein (Köhler and Milstein, 1976, Eur. J. Immunol., 6:511-519).

In a preferred embodiment, a detecting agent as described herein above may comprise a detectable label. Any suitable label, which can be attached to the detecting agent may be used. In one preferred embodiment the detectable label is covalently or non-covalently attached to the detecting agent. Examples of labels that may be attached to the detecting agent include e.g. fluorescent dyes such as e.g. Cyanine dyes, e.g. Cyanine 3, Cyanine 5 or Cyanine 7, Alexa Fluor dyes, e.g. Alexa 594, Alexa 488 or Alexa 532, fluorescein family dyes, R-Phycoerythrin, Texas Red and rhodamine. Detecting agents may also be labeled with enzymes such as e.g. horseradish peroxidase, alkaline phosphatase or beta-lactamase, radioisotopes such as e.g. ³H, ¹⁴C, ³²P, ³³P, ³⁵S or ¹²⁵I or metal such as e.g. gold. In another preferred embodiment the detecting agent may also be detected by a secondary detecting agent comprising a label as described above. Preferably a secondary detecting agent is capable of specifically binding to the above described detecting agent. In a particularly preferred embodiment a secondary detecting agent is an antibody.

In some embodiments the amount of H3T6ph may e.g. be detected in methods involving histological or cell-biological procedures. In some embodiments, visual techniques, such as light microscopy or immunofluoresence microscopy, or flow cytometry or luminometry may be used. In a preferred embodiment H3T6ph is detected by immunohistochemistry.

In further preferred embodiments determining the amount of H3T6ph in the sample from the subject may be performed alongside measuring or determining the amount of other compounds or factors, such as e.g. determining the level of prostate-specific antigen (PSA) or Insulin-like Growth Factor-1 (IGF-1) in the same sample or in a different sample from the same subject.

The term "detecting cancer" as used herein means that the presence of a cancerous disease or disorder may be identified in a subject or in a sample from a subject. Preferably, said subject is previously not known to suffer from cancer. In one preferred embodiment the subject is suspected to suffer from cancer. The term "detecting cancer" as used herein is thus meant to encompass "diagnosing cancer". The terms "detecting cancer" and "diagnosing cancer" may be used interchangeably.

The determination or identification of a cancerous disease or disorder may e.g. be accomplished by comparing the amount of H3T6ph determined in the sample from the subject to the amount of the respective compound being present in a control as herein described below.

The term "grading cancer" as used herein refers to classifying the cancer by determining certain features of the cancer, such as e.g. its aggressiveness and its prognosis.

The inventors of the present invention have surprisingly found that elevated levels of protein kinase C ß1, H3T6ph, H3K4me1, H3K4me2 and H3K4me3 correlate with high Gleason scores in human prostate cancer. Therefore, in one preferred embodiment "grading cancer", preferably prostate cancer, in the context of the present invention may be performed by correlating the amount of H3T6ph determined in the sample from the subject to cancer grade, preferably prostate cancer grade.

Correlating the amount of H3T6ph determined in the sample from the subject to cancer grade may for example be achieved by comparing the amount of H3T6ph determined in the sample to previously determined values for the amount of H3T6ph which was determined in one or more control sample(s) from subjects known to suffer from cancer and which were assigned to different tumor grades, preferably to different Gleason scores.

The term "prognosticating cancer" as used herein refers to the prediction of the course or outcome of a diagnosed or detected cancerous disease, e.g. during a certain period of time, e.g. during treatment or after treatment. The term may also refer to a determination of chance of survival or recovery from the cancerous disease, as well as to a prediction of the expected survival time of a subject.

For example, the amount of H3T6ph may be determined in a sample from a subject at a given point of time and compared to the respective amount of H3T6ph determined in a sample from the same subject at a later point of time, wherein an increase or decrease in the amount of H3T6ph indicates cancer cell proliferation or cancer regression respectively. Such an approach may e.g. be used during cancer treatment, e.g. during application of anti-cancer medication to a subject suffering from cancer.

Thus in one preferred embodiment the methods according to the invention may be used to monitor the efficacy of cancer treatment in vitro. The efficacy of cancer treatment may e.g. be monitored by detecting the amount of H3T6ph in different samples from a subject that were provided over a given period of time while the subject from which the samples were derived was subjected to anti-cancer treatment. An increase or decrease in the amount of H3T6ph in samples provided from the subject over a given period of time may then indicate the efficacy of anti-cancer treatment.

In a preferred embodiment the above method further comprises the step of comparing the amount of histone H3 phosphorylated at threonine 6 (H3T6ph) determined in the sample from the subject to the amount of the respective compound determined in a control. The term "respective compound " means that the amount of each compound in the sample is compared to the amount of the respective compound in the control, i.e. e.g. the amount of histone H3 phosphorylated at threonine 6 (H3T6ph) in the sample is compared to the amount of histone H3 phosphorylated at threonine 6 (H3T6ph) in the control.

In one preferred embodiment the control is a sample from a healthy subject. In a preferred embodiment a higher amount of H3T6ph determined in the sample from the subject in comparison to the control indicates the presence of cancer in the subject. The term "higher amount" means that the amount of H3T6ph determined in the sample from the subject is preferably at least 2 fold, at least 3 fold, at least 4 fold, at least 5 fold, at least 10 fold, at least 20 fold, at least 30 fold, at least 40 fold, at least 50 fold, at least 60 fold, at least 70 fold, at least 80 fold, at least 90 fold, at least 100 fold, at least 500 fold, at least 1000 fold or at least 10000 fold higher than in the control.

In some other preferred embodiments, preferably in cases where grading of the cancer is desired, the control may also be a sample derived from a subject known to suffer from cancer, i.e. a subject that has been independently diagnosed with cancer, preferably with prostate cancer or breast cancer. Preferably a control derived from a subject known to suffer from cancer has previously been subjected to cancer grading. Thus, in one preferred embodiment the amount of histone H3 phosphorylated at threonine 6 (H3T6ph) determined in the sample from the subject in comparison to the control may indicate the grade of the cancer present in the subject.

In one preferred embodiment the amount of H3T6ph in the control may be determined in parallel to the amount of H3T6ph in the sample from the subject.

In another preferred embodiment the control may be a predetermined value. Such a value may e.g. be based on the results of previous experiments determining the amount of H3T6ph, in one or more samples from a healthy subject or a subject known to suffer from cancer, preferably prostate cancer or breast cancer. In some embodiments a predetermined value may be derivable from a database.

In one preferred embodiment the amount of H3T6ph may be compared to more than one control, e.g. to 2, 3, 4, 5, 6, 7, 8, 9, 10, 20, 30, 40, 50, 60, 70, 80, 90, 100 or more than 100 controls.

. In a preferred embodiment determining the amount of protein kinase beta 1 and/or 2 protein in the sample from the subject is performed alongside determining the amount of histone H3 phosphorylated at threonine 6 (H3T6ph), the amount of histone H3 monomethylated at lysine 4 (H3K4me1), the amount of histone H3 dimethylated at lysine4 (H3K4me2) and the amount of histone H3 trimethylated at lysine 4 (H3K4me3) in the same sample or in a further sample from the same subject.

The sample from the subject used in the methods according to the invention is separated from the body of the subject. The sample may be solid or liquid. In a preferred embodiment the sample from the subject is a body fluid or tissue sample, preferably a prostate or breast tissue sample. Preferably, the tissue sample is derived from a cancer tissue, most preferably a prostate or breast cancer tissue. Tissue samples may e.g. be fresh or frozen tissue samples or fixed paraffin embedded samples. In one preferred embodiment the sample may be a biopsy or resection sample.

Preferably, the body fluid is blood, plasma, urine, saliva, serum, semen, prostate fluid or seminal fluid.

In some embodiments, a liquid sample may be enriched for cells of interest, e.g. prostate cells. Enrichment may be performed by any method known to the skilled person. Enrichment may e.g. be achieved by using a solid support, e.g. a column, coated with a specific antibody, such as e.g. a prostate specific antibody. Alternatively, enrichment may e.g. also be achieved by using filtration methods or by immobilizing specific aptamers on a microfluidic channel and pumping the liquid sample through the device.

In a further aspect the present invention also relates to a diagnostic kit for detecting, grading, and/or prognosticating cancer comprising a detecting agent specific for histone H3 phosphorylated at threonine 6 (H3T6ph), wherein the detecting agent is an antibody, an aptamer or an oligonucleotide probe.

Preferably, the diagnostic kit according to the invention contains one or more detecting agents specific for histone H3 phosphorylated at threonine 6 (H3T6ph). If the diagnostic kit according to the invention comprises more than one detecting agent, said detecting agents are preferably each specific for histone H3 phosphorylated at threonine 6 (H3T6ph). Suitable detecting agents have been described herein above. The diagnostic kit may further comprise additional components or reagents that may be suitable for performing the methods according to the invention, such as e.g. buffers or controls. The components contained in the diagnostic kit may be comprised in one or more containers. The diagnostic kit according to the present invention may also comprise an instruction leaflet, which indicates how to use the diagnostic kit and its components.

The present invention in a further aspect also relates to the use of a detecting agent specific for histone H3 phosphorylated at threonine 6 (H3T6ph), detecting, grading and/or prognosticating cancer in a sample from a subject, wherein the detecting agent is an antibody, an aptamer or an oligonucleotide probe. Preferably, said use in vitro.

Suitable detecting agents are described herein above.

In another aspect the present invention relates to the diagnostic kit according to the invention or the use of a detecting agent specific for histone H3 phosphorylated at threonine 6 (H3T6ph) for detecting, grading and/or prognosticating cancer in a sample from a subject, wherein the detecting agent is an antibody, an aptamer or an oligonucleotide probe.

Examples of suitable antibodies, aptamers and oligonucleotide probes have been described herein above.

In a further aspect the present invention relates to the methods according to the invention, the diagnostic kit according to the invention, and/or the use according to the invention, wherein the cancer is selected from the group consisting of prostate cancer, breast cancer, lung cancer, pancreatic cancer, neuroblastoma, melanoma, brain cancer, kidney cancer, bladder cancer, ovarian cancer, blood cancer and colon cancer. In a particularly preferred embodiment the cancer is prostate cancer.

The present invention will now be described with respect to some of its specific examples.

### Examples

### Material and Methods

### Plasmids

The following plasmids were described previously: pGEX-4T1-H3 1-15, pGEX-4T1-H3 16-30, pGEX-4T1-H3 29-44, pET-GEX H3 1-135, and pLenti6-miRNA-control³. GST-H3 1-135 T6A was provided by J.M.G. Higgins. To construct GST H3 1-15 T6A the corresponding cDNA fragment was cloned into pGEX-4T1. To construct pLenti6-miRNA-PKCβI, the DNA corresponding to miRNA-PKCβI (5'-TGCTGTTACGTAGGGATCTGACAGGCGTTTTGG CCACTGACTGACGCCTGTCATCCCTACGTAA-3' (SEQ IDNO:6) and 5'-CCTGTTACGTAGGGATGACAGGCGTCAGTCAGTGGCCAAAACGCCTGTCA GATCCCTACGTAAC-3') (SEQ ID NO:5) was cloned into pLenti6/V5-DEST according to the manufacturer's instructions (Invitrogen).

### Chromatin immunoprecipitation

ChIP and Re-ChIP experiments were performed as described³. LNCaP cells were cultured for 15min (ChIP) or 210 min (Re-ChIP) in the presence or absence of 1 x 10⁻⁸ M R1881 as indicated. Three days before harvesting, cells were transfected with siRNA (control for PKCβI: 5'-CAUUCCUAUGCGAGACGUAUGUAAA-3'(SEQ IDNO 3); PKCβI: 5'-CAUUGUCCUCGUAAGAGAUGCUAAA-3' (SEQ ID NO 4) (Fig. 3 b, c, f-i); control for PRK1: 5'-GAAAGUCCUAGAUCCACACGCAAAU-3' (SEQ ID NO 14); PRK1: 5'-GAACAUGAUCCAGACCUACAGCAAU-3' (SEQ ID NO: 15) (Fig. 3 d, e); Invitrogen) using RNAifect (Qiagen) following the manufacturer's instructions. Immunoprecipitation was performed with specific antibodies (α-AR # 06-680 lot 33529 (Upstate Biotechnology), α-H3K4me1 # ab8895 lot 535654, α-H3T6ph # ab14102 lot 481643, α-H3 # ab1791 lot 172452, α-PKCβI S660ph # ab23513 lot 596904 (Abcam), α-PKCβI (C-16) # sc-209 lot 481643, (Santa Cruz), α-H3K4me2 # cs-035-100 lot A391001 (Diagenode), α-PRK1³, and α-LSD1²) on protein A-Sepharose 4B (GE-Healthcare). For PCR, 2 µl out of 50 µl DNA extract was used. PCR primers for *FKBP5 (+351*/*+443), KLK2* (-343/- 90), *SCN1A, SCN2A, GAPDH,* and *U6* were described previously ^{1,2,6,11}.

### Western blot analysis

Experiments were performed as described². Western blots were decorated as indicated.

### Cell proliferation assay

Experiments were performed as described². pLenti6-miRNA-control and pLenti6-miRNA-PKCβI were used to produce recombinant lentiviruses to infect LNCaP cells as described¹⁵. The infected cells were cultured for 72 hours in medium containing 10% double-stripped FCS. 1x10⁴ cells were plated in a 96-well plate in the presence or absence of 1 x 10⁻⁹ M R1881. The cell proliferation Elisa BrdU Colorimetric Assay (Roche) was performed according to the manufacturer's instructions. The figure shows the increase of proliferation in the presence versus absence of R1881. The experiments were performed in hexaplicate.

### Quantitative RT-PCR and statistical analysis

Quantitative RT-PCR and statistical analysis were performed as described². The primers for *KLK2* and *FKBP5* were described previously^{6,11}.

### In vitro kinase assay

The kinase assays were performed as described³. Ten µg of either GST-H3 mutants (Fig. 1b), or 1 µg of nucleosomes (Fig. 1a) purified from HeLa cells¹⁶ were incubated with the amount corresponding to 12 pmol/min⁻¹ purified recombinant PKC (ProQinase) at 30°C in kinase buffer containing 60 mM Hepes-NaOH pH 7.5, 3 mM MgCl₂, 3 mM MnCl₂ and either 10 mM ATP (Fig. 1a) or 5 µCi [γ-³²P]ATP (Fig. 1b). The reaction mixture was analysed by Western blotting using antibodies as indicated (Fig. 1a) or SDS-PAGE followed by autoradiography (Fig. 1b).

### Immunohistochemistry

Stainings were performed using a protocol¹⁷ for antigen retrieval and indirect immunoperoxidase. α-AR (# sc-7305 lot E171; Santa Cruz), α-PKCβI (# sc-209 lot 481643; Santa Cruz), α-H3T6ph (# ab14102 lot 481643; Abcam), and α-H3K4me2 (# ab7766-100, lot 56290; Abcam) antibodies were used at a dilution of 1:75, 1:1000, 1:200, and 1:1000, respectively. Immunoreactions were performed with the universal vectastain ABC kit according to the manufacturer's instructions (Vector Laboratories).

### Tissue microarrays and statistical analysis

Clinical data of patients and procedures for generating the tissue microarrays were described previously¹⁷. Tissue microarrays were prepared from formalin-fixed, paraffin-embedded tissue specimens of 154 prostates selected from the archival files of the Institute of Pathology, University of Bonn Medical School. All tumour samples were surgically obtained from patients who had undergone radical retropubic prostatectomy in two surgical centres between 1995 and 2002 for clinically organ confined prostate cancer (preoperative staging </= cT2, cNo, cMo). Patients who had received prior hormonal therapy, chemotherapy, or radiation therapy were excluded from the study. All cases were re-evaluated by a panel of experienced pathologists for histopathological staging according to the UICC TNM-system¹⁸, rescored according to the Gleason scoring system¹⁹ and subsequently followed-up between 21 to 128 months (median 40.24 months). Three different tissue cores representing the lowest and highest Gleason grades within a single tumour were arrayed from formalin-fixed, paraffin-embedded tissue blocks using a manual device (Beecher Instruments). 4 µm paraffin sections were cut from every TMA and used for subsequent immunohistochemical analyses within one week. Statistical analysis was performed with the Mann-Whitney U-Test using the SPSS 12.0 program (SPSS Inc.) and by calculating the two-tailed Spearman Rank correlation coefficient. The number of cases (n) analysed per Gleason score (Gs) were: Gs 3 (n=5); Gs 4 (n=12); Gs 5 (n=14); Gs 6 (n=38); Gs 7 (n=24); Gs 8 (n=27); Gs 9 (n=20); Gs 10 (n=14). Normal prostate specimens (n=25).

### Demethylase assay and mass spectrometry analysis

The demethylation assays were performed essentially as described^{1, 20}. One µg peptides corresponding to the H3 tail residues 1-20 carrying either one, two, or three methyl groups at K4 in the absence or presence of a phosphorylated T6 were incubated with 5 µg bacterially expressed and purified His-LSD1, or 5 µg baculovirus expressed and purified GST-JARID1B and GST as indicated. Peptides were incubated for 5 hours at 37°C in demethylation buffer containing 50 mM Tris HCl pH 7.5, 50 mM KCl, 5 mM MgCl₂ or 50 mM HEPES-KOH pH 8.0, 2 mM ascorbate, 100 µM Fe(NH₄)₂(SO₄)₂, 1 mM α-ketoglutarate^{1, 20}. The crude demethylase reactions were diluted 1:10 in a saturated CHCA solution (50% acetonitril, 0.1%TFA) and spotted onto a MALDI-target plate. For each reaction 2000 spectra were recorded and analysed using the Data Explorer Software™.

### Example 1 - H3T6 is phosphorylated by PKCßI

To identify putative H3T6 kinases, *in vitro* kinase screens were performed by incubating nucleosomes purified from HeLa cells with 97 recombinantly expressed, purified kinases (Fig. 5). Western blot analysis performed with antibody specific to phosphorylated H3T6 (aH3T6ph) demonstrates that only PKCα, β1 and β2 phosphorylate nucleosomes at H3T6 (Fig. 1a). PKCα,β1 and β2 specifically target H3T6 but not threonine 3, serine 10, or threonine 11 of histone H3 (H3T3, H3S10, H3T11, respectively) (Fig. 1a). Specificity of the antibodies was verified (Fig. 6). Western blot analysis performed with α-PKCα, β1 or β2 antibodies shows that in LNCaP human prostate cancer cells only PKCβI is expressed (Fig.7). Therefore, all further studies were continued with PKCβI. To corroborate in an independent approach that PKCβI exclusively phosphorylates H3T6, PKCβI was incubated with recombinant, purified GST-H3 or GST control proteins in the presence of [γ-³²P]ATP. In these experiments only full-length histone H3 (H3 1-135) and a H3 fragment spanning amino acid residues 1 to 15 (H3 1-15), but not H3 16-30 or H3 29-44, are phosphorylated by PKCβI (Fig. 1b). Furthermore, mutation of threonine 6 to alanine in either H3 1-15 (H3 1-15 T6A) or in full length H3 (H3 1-135 T6A) abolishes phosphorylation, demonstrating that PKCβI targets H3T6 only (Fig. 1b). Taken together, said data identify PKCβI as the first H3T6 kinase.

### Example 2- phosphorylation of H3T6 blocks the demethylation of H3K4 methyl marks

Demethylation assays were performed with di-, and monomethyl H3K4 peptides either unmodified at T6 (H3K4me2 and H3K4me1) or phosphorylated at T6 (H3K4me2T6ph and H3K4me1T6ph) and recombinant, purified LSD1 (Fig. 2 a, b and Fig. 8). Following the demethylation assay, the peptides were analysed by mass spectrometry. The robust demethylation of H3K4me2 observed in the presence of LSD1 (Fig. 2 a, compare panels 1 and 2) is completely blocked when the peptides are phosphorylated at T6 (Fig. 2 a, compare panels 3 and 4). Likewise, demethylation of H3K4me1 by LSD1 is completely abrogated in the presence of phosphorylated T6 (Fig. 2 b, panels 3 and 4). Since LSD1 only removes mono- and dimethyl marks^{1, 2}, it was tested whether phosphorylation at H3T6 also blocks demethylation of tri-, and dimethyl H3K4 (H3K4me3 and H3K4me2) by members of the KDM5 family of JMJC domain-containing demethylases such as JARID1B/KDM5B⁹. Recombinant, purified JARID1B (Fig. 9) efficiently demethylates H3K4me3 and H3K4me2 peptides (Fig. 10 a, b, compare panels 1 and 2). In contrast, demethylation of tri- and dimethyl H3K4 peptides phosphorylated at T6 (H3K4me3T6ph and H3K4me2T6ph) is severely impaired (Fig. 10 a, b, compare panels 3 and 4). These results demonstrate that phosphorylation of H3T6 blocks demethylation of H3K4 methyl marks *in vitro.*

### Comparative Example 3- PKCβI and LSD1 form a complex on chromatin in a ligand-dependent manner

Western blot analysis performed with anti-PKC α, β1 or β2 antibodies indicates that only PKCβI is expressed in LNCaP cells (Fig. 7). Therefore, it was investigated whether PKCβI is present at AR target genes and phosphorylates H3T6 *in vivo.* To analyse first whether PKCβI binds to promoters of AR target genes, LNCaP cells were subjected to chromatin immunoprecipitation (ChIP) in the presence or absence of the AR agonist R1881. PKCβI specifically associates with the androgen response elements (AREs) located in the promoters of the *KLK2¹⁰* and *FKBP5¹¹* genes (Fig. 3a, upper panel). Association of PKCβI with AR target promoters is specific since DNA from neither *GAPDH* nor U6 promoters is enriched (Fig. 3a, upper panel). PKCβI binds to chromatin in a ligand-independent manner similar to LSD1 (ref² and see Fig. 3a, upper panel). In comparison, Protein-kinase-C-related kinase 1 (PRK1) and AR bind chromatin only in a ligand-dependent manner. To investigate whether PKCβI and AR are present in the same complex on the *KLK2* and *FKBP5* promoters, R1881-treated LNCaP cells were subjected to sequential chromatin immunoprecipitation (Re-ChIP), first with α-AR and then with α-PKCβI antibody. PKCβI and AR form a complex on chromatin upon addition of ligand (Fig. 3a, lower panel). Similarly, it was investigated whether PKCβI and LSD1 are present in the same complex on the *KLK2* and *FKBP5* promoters. Re-ChIP first with α-PKCβI and then with α-LSD1 antibody demonstrates that PKCβI and LSD1 form a complex on chromatin in a ligand-independent manner (Fig. 3a, lower panel).

### Example 4- H3T6ph is a novel chromatin mark for transcriptional activation

Next, it was invetigated whether PKCβI phosphorylates H3T6 at promoters of AR-regulated genes *in vivo.* LNCaP cells, cultured in the presence or absence of R1881, were transfected with either an unrelated control siRNA or a siRNA directed against PKCβI, and then subjected to ChIP with α-H3T6ph antibody. Importantly, addition of ligand results in phosphorylation of H3T6 at the AREs of the *KLK2* and *FKBP5* promoters (Fig. 3b, c). Androgen-induced phosphorylation at H3T6 is PKCβI-dependent since it is blocked by knockdown of PKCβI. PKCβI depletion is specific and does not affect the levels of endogenous AR (Fig. 3c, right panel). PKCβI depletion does neither affect ligand-dependent recruitment of AR nor the presence of LSD1 on AR-regulated genes (Fig. 3b, c). Since PKCβI is present on the promoter of AR target genes irrespective of the presence or absence of ligand, whereas H3T6 phosphorylation is only observed in the presence of R1881, it was investigated whether PKCβI is phosphorylated and thereby activated in an androgen-dependent manner. Active PKCβI is characterised by phosphorylated serine 660¹² (S660ph). ChIP assays using α-PKCβI S660ph antibody show androgen-induced phosphorylation at S660 of PKCβI at the *KLK2* and *FKBP5* promoters (Fig. 3b, c). Taken together, these data demonstrate androgen-dependent activation of PKCβI and phosphorylation of H3T6 by *PKCβI in vivo.*

### Example 5- PKCβI induced phosphorylation at H3T6 is controlled by PRK1

It was recently shown by the inventors that PRK1 is a gatekeeper for regulation of AR target gene expression³. PRK1 controls multiple processes during AR-dependent transcription, such as transition from the RNA polymerase II pre-initiation to the initiation complex and demethylation at lysine 9 of histone H3 (H3K9)³. Therefore, it was tested whether in the presence of ligand PKCβI and PRK1 are present in the same complex at promoters of AR target genes. Re-ChIP analysis first with α-PKCβI and then with α-PRK1 antibody demonstrates that both proteins form a complex on the *KLK2* and *FKBP5* promoters upon addition of ligand (Fig. 3a, lower panel). It was next investigated whether PRK1 regulates PKCβI activity. LNCaP cells, grown in the presence or absence of R1881, were transfected with either an unrelated control siRNA or a siRNA directed against PRK1 and then subjected to ChIP. Ligand-dependent activation of PKCβI is blocked by knockdown of PRK1 (Fig. 3d, e). Consequently, ligand-dependent phosphorylation of H3T6 at the AREs of the *KLK2* and *FKBP5* promoters is abolished (Fig. 3d, e). PRK1 depletion is specific and does neither affect the levels of endogenous AR (Fig. 3e, right panel) nor the presence of PKCβI on AR target promoters (Fig. 3d, e). In summary, these data demonstrate that PKCβI phosphorylates H3T6 on chromatinized promoters of AR target genes in an androgen- and PRK1-dependent manner.

### Example 6- PKCβI induced phosphorylation at H3T6 blocks demethylation of methylated H3K4me2 and H3K4me1 during AR induced gene expression

It was analysed whether PKCβI protects demethylation of H3K4 at androgen regulated promoters *in vivo.* LNCaP cells were cultivated in the presence or absence of R1881, transfected with either an unrelated control siRNA or a siRNA directed against PKCβI and subjected to ChIP. As shown in Fig. 3f and 3g, levels of H3K4me2 and H3K4me1 observed at the *KLK2* and *FKBP5* promoters in the absence of ligand are increased upon the addition of R1881, which is in agreement with previous data¹³. Furthermore, the ligand-dependent increase in H3K4me2 and H3K4me1 correlates with R1881-induced phosphorylation of H3T6 by PKCβI. Knockdown of PKCβI blunts ligand-induced phosphorylation of H3T6 and reduces H3K4me2 and H3K4me1 levels to those observed in the absence of R1881. Antibody recognition of H3K4 methyl marks is not altered by phosphorylation at H3T6 (Fig. 11). Together, said observations show that phosphorylation of H3T6 by PKCβI interferes with the removal of H3K4 methyl marks by LSD1 *in vivo.* Elevated levels of H3K4me2 and H3K4me1 are observed upon PKCβI knockdown in the absence of R1881 indicating androgen-independent functions of PKCβI beyond phosphorylation of H3T6 (Fig. 3f, g). Next, it was tested whether PKCβI signalling also occurs on genes such as *SCN1A* or *SCN2A* where LSD1 removes active H3K4 methyl marks to silence gene expression¹. ChIP assays performed in LNCaP and HeLa cells show that opposite to AR-activated genes, PKCβI does not associate with LSD1-silenced genes and consequently H3T6 is not phosphorylated (Fig. 3h, i and Fig. 12a, b).

### Example 7- PKCβI plays an important role in the control of AR-dependent tumour cell growth

To assess the physiological role of PKCβI, the levels of PKCβI, H3T6ph and H3K4me2 were analysed by immunostaining a panel of 25 normal human prostates and 154 prostate carcinomas on tissue microarrays (Fig. 4 a-d and f). Quantification of immunoreactivity by scoring staining intensity and percentage of positive carcinoma cells¹⁴ reveals that high PKCβI, H3T6ph, and H3K4me2 levels significantly correlate with high Gleason scores and indicate aggressive biology of the tumours (Fig. 4b-d and f). To examine whether PKCβI regulates tumour cell proliferation, androgen-dependent proliferation of LNCaP cells was quantified upon PKCβI knockdown by RNAi. Androgen-induced proliferation of LNCaP cells is severely reduced by PKCβI knockdown when compared to control cells (Fig. 4e), emphasising the important role of PKCβI in the control of AR-dependent tumor growth.

### Literature

1. Shi, Y. et al. Histone demethylation mediated by the nuclear amine oxidase homolog LSD1. Cell 119, 941-953. (2004).
2. Metzger, E. et al. LSD1 demethylates repressive histone marks to promote androgen-receptor-dependent transcription. Nature 437, 436-439. (2005).
3. Metzger, E. et al. Phosphorylation of histone H3 at threonine 11 establishes a novel chromatin mark for transcriptional regulation. Nat. Cell Biol. 10, 53-60. (2008).
4. Strahl, B. D. & Allis, C. D. The language of covalent histone modifications. Nature 403, 41-45. (2000).
5. Klose, R.J., Kallin, E.M. & Zhang, Y. JmjC-domain-containing proteins and histone demethylation. Nat. Rev. Genet. 7, 715-727. (2006).
6. Wissmann, M. et al. Cooperative demethylation by JMJD2C and LSD1 promotes androgen receptor-dependent gene expression. Nat. Cell Biol. 9, 347-353. (2007).
7. Yang, M. et al. Structural basis of histone demethylation by LSD1 revealed by suicide inactivation. Nat. Struct. Mol. Biol. 14, 535-539. (2007).
8. Forneris, F., Binda. C., Adamo, A., Battaglioli, E. & Mattevi, A. Structural basis of LSD1-CoREST selectivity in histone H3 recognition. J. Biol. Chem. 282, 20070-20074. (2007).
9. Metzger, E. & Schüle R. The expanding world of histone lysine demethylases. Nat. Struct. Mol. Biol. 14, 252-254. (2007).
10. Kang, Z., Pirskanen, A., Janne, O. A. & Palvimo, J. J. Involvement of proteasome in the dynamic assembly of the androgen receptor transcription complex. J Biol. Chem. 277, 48366-48371. (2002).
11. Magee, J.A. et al. Direct, androgen receptor mediated regulation of the FKBP5 gene via a distal enhancer element. Endocrinology 147, 590-598. (2005).
12. Keranen L.M., Dutil E.M. & Newton A.C. Protein kinase C is regulated in vivo by three functionally distinct phosphorylations. Curr. Biol. 5, 1394-1403. (1995).
13. Kang, Z., Jänne O.A. & Palvimo J.J. Coregulator recruitment and histone modifications in transcriptional regulation by the androgen receptor. Mol. Endocrinol. 18, 2633-2648. (2004).
14. Kononen J. et al. Tissue microarrays for high-throughput molecular profiling of tumour specimens. Nat. Med. 4, 844-847. (1998).
15. Wiznerowicz, M. & Trono, D. Conditional suppression of cellular genes: lentivirus vector-mediated drug-inducible RNA interference. J. Virol. 77, 8957-8961. (2003).
16. O'Neill, T. E., Roberge, M. & Bradbury, E. M. Nucleosome arrays inhibit both initiation and elongation of transcripts by bacteriophage T7 RNA polymerase. J. Mol. Biol. 223, 67-78. (1992).
17. Kahl, P. et al. Androgen receptor coactivators lysine-specific histone demethylase 1 and four and a half LIM domain protein 2 predict risk of prostate cancer recurrence. Cancer Res. 66, 11341-11347. (2006).
18. Schroder, F.H. et al. The TNM classification of prostate cancer. Prostate Suppl. 4, 129-138. (1992).
19. Hsing, A.W., Tsao L. & Devesa S.S. International trends and patterns of prostate cancer incidence and mortality. Int. J. Cancer 85, 60-67. (2000).
20. Tsukada, Y. I. et al. Histone demethylation by a family of JmjC domain-containing proteins. Nature 435, 811-816. (2005).
21. Dai, J. et al. The kinase haspin is required for mitotic histone H3 Thr 3 phosphorylation and normal metaphase chromosome alignment. Genes Dev. 19, 472-488. (2005).

## Claims

1. A method for detecting, grading and/or prognosticating cancer comprising the step of determining in a sample from a subject the amount of histone H3 phosphorylated at threonine 6 (H3T6ph), wherein said method is performed in vitro.

2. The method according to claim 1, further comprising the step of comparing the amount of histone H3 phosphorylated at threonine 6 (H3T6ph) determined in the sample from the subject to the amount of histone H3 phosphorylated at threonine 6 (H3T6ph) determined in a control.

3. The method according to claim 1 or 2, wherein the sample from the subject is a body fluid or tissue sample, preferably a prostate or breast tissue sample.

4. The method according to claim 3, wherein the body fluid is selected from the group of blood, plasma, urine, saliva, serum, semen, prostate fluid or seminal fluid.

5. The method according to any of claims 1 to 4, wherein the cancer is selected from the group consisting of prostate cancer, breast cancer, lung cancer, pancreatic cancer, neuroblastoma, melanoma, brain cancer, kidney cancer, bladder cancer, ovarian cancer, blood cancer and colon cancer.

6. A diagnostic kit for detecting, grading and/or prognosticating cancer comprising a detecting agent specific for histone H3 phosphorylated at threonine 6 (H3T6ph), wherein the detecting agent is an antibody, an aptamer or an oligonucleotide probe.

7. The diagnostic kit according to claim 6, wherein the cancer is selected from the group consisting of prostate cancer, breast cancer, lung cancer, pancreatic cancer, neuroblastoma, melanoma, brain cancer, kidney cancer, bladder cancer, ovarian cancer, blood cancer and colon cancer.

8. Use of a detecting agent specific for histone H3 phosphorylated at threonine 6 (H3T6ph) for detecting, grading and/or prognosticating cancer in a sample from a subject, wherein the detecting agent is an antibody, an aptamer or an oligonucleotide probe.

9. The use according to claim 8, wherein the cancer is selected from the group consisting of prostate cancer, breast cancer, lung cancer, pancreatic cancer, neuroblastoma, melanoma, brain cancer, kidney cancer, bladder cancer, ovarian cancer, blood cancer and colon cancer.

## Patentansprüche

1. Eine Methode zur Detektion, Einstufung und/oder Prognose von Krebs umfassend den Schritt der Bestimmung der Menge von Histon H3, das am Threonin 6 phosphoryliert ist (H3T6ph), in einer Probe von einem Subjekt, wobei besagte Methode in vitro durchgeführt wird.

2. Die Methode gemäß Anspruch 1, weiterhin umfassend den Schritt des Vergleichens der Menge von Histon H3, das am Threonin 6 phosphoryliert ist (H3T6ph), welche in der Probe von dem Subjekt bestimmt wurde, mit der Menge von Histon H3, das am Threonin 6 phosphoryliert ist (H3T6ph), welche in einer Kontrolle bestimmt wurde.

3. Die Methode gemäß Anspruch 1 oder 2, wobei die Probe von dem Subjekt eine Körperflüssigkeit oder eine Gewebeprobe, bevorzugt eine Prostata- oder Brustgewebeprobe, ist.

4. Die Methode gemäß Anspruch 3, wobei die Körperflüssigkeit ausgewählt ist aus der Gruppe aus Blut, Plasma, Urin, Speichel, Serum, Samen, Prostataflüssigkeit oder Samenflüssigkeit.

5. Die Methode gemäß einem der Ansprüche 1 bis 4, wobei der Krebs ausgewählt ist aus der Gruppe bestehend aus Prostatakrebs, Brustkrebs, Lungenkrebs, Pankreaskrebs, Neuroblastom, Melanom, Gehirnkrebs, Nierenkrebs, Blasenkrebs, Ovarialkrebs, Blutkrebs und Darmkrebs.

6. Ein diagnostisches Kit zur Detektion, Einstufung und/oder Prognose von Krebs umfassend ein Detektionsagens, das spezifisch für Histone H3, das am Threonin 6 phosphoryliert ist (H3T6ph), ist, wobei das Detektionsagens ein Antikörper, ein Aptamer oder eine Oligonukleotidsonde ist.

7. Das diagnostische Kit gemäß Anspruch 6, wobei der Krebs ausgewählt ist aus der Gruppe bestehend aus Prostatakrebs, Brustkrebs, Lungenkrebs, Pankreaskrebs, Neuroblastom, Melanom, Gehirnkrebs, Nierenkrebs, Blasenkrebs, Ovarialkrebs, Blutkrebs und Darmkrebs.

8. Verwendung eines Detektionsagens, das spezifisch für Histon H3, das am Threonin 6 phosphoryliert ist (H3T6ph), ist, zur Detektion, Einstufung und/oder Prognose von Krebs in einer Probe von einem Subjekt, wobei das Detektionsagens ein Antikörper, ein Aptamer oder eine Oligenukleotidsonde ist.

9. Die Verwendung gemäß Anspruch 8, wobei der Krebs ausgewählt ist aus der Gruppe bestehend aus Prostatakrebs, Brustkrebs, Lungenkrebs, Pankreaskrebs, Neuroblastom, Melanom, Gehirnkrebs, Nierenkrebs, Blasenkrebs, Ovarialkrebs, Blutkrebs und Darmkrebs.

## Revendications

1. Méthode pour détecter un, évaluer le stade d'un et/ou, établir le pronostic d'un cancer, comprenant l'étape de détermination dans un échantillon provenant d'un sujet de la quantité d'histone H3 phosphorylée au niveau de la thréonine 6(H3T6ph), ladite méthode étant mise en oeuvre in vitro.

2. Méthode suivant la revendication 1, comprenant en outre l'étape de comparaison de la quantité d'histone H3 phosphorylée au niveau de la thréonine 6(H3T6ph) déterminée dans l'échantillon provenant du sujet avec la quantité d'histone H3 phosphorylée au niveau de la thréonine 6(H3T6ph) déterminée dans un témoin.

3. Méthode suivant, la revendication 1 on 2, dans laquelle l'échantillon provenant du sujet est un fluide corporel ou échantillon de tissu, de préférence un échantillon de tissu de la prostate ou du sein.

4. Méthode suivant la revendication 3, dans laquelle le fluide corporel est choisi dans le groupe comprenant le sang, le plasma, l'urine, la salive, le sérum, le sperme, le fluide prostatique ou le liquide séminal.

5. Méthode suivant l'une quelconque des revendications 1 à 4, dans laquelle le cancer est choisi dans le groupe consistant en le cancer de la prostate, le cancer du sein, le cancer du poumon, le cancer du pancréas, un neuroblastome, un mélanome, le cancer du cerveau, le cancer des reins, le cancer de la vessie, le cancer des ovaires, un cancer du sang et le cancer du côlon.

6. Kit de diagnostic pour détecter un, évaluer le stade d'un et/ou établir le pronostic d'un cancer, comprenant un agent de détection spécifique de l'histone H3 phosphorylée au niveau de la thréonine 6(H3T6ph), dans lequel l'agent de détection est un anticorps, un aptamère ou une sonde oligonucléotidique.

7. Kit de diagnostic suivant la revendication 6, dans lequel le cancer est choisi dans le groupe consistant en le cancer de la prostate, le cancer du sein, le cancer du poumon, le cancer du pancréas, un neuroblastome, un mélanome, le cancer du cerveau, le cancer des reins, le cancer de la vessie, le cancer des ovaires, un cancer du sang et le cancer du côlon.

8. Utilisation d'un agent de détection spécifique de l'histone H3 phosphorylée au niveau de la thréonine 6 (H3T6ph) pour détecter un, évaluer le stade d'un et/ou établir le pronostic d'un cancer dans un échantillon provenant d'un sujet, dans laquelle l'agent de détection est un anticorps, un aptamère ou une sonde oligonucléo-tidique.

9. Utilisation suivant la revendication 8, dans laquelle le cancer est choisi dans le groupe consistant en le cancer de la prostate, le cancer du sein, le cancer du poumon, le cancer du pancréas, un neuroblastome, un mélanome, le cancer du cerveau, le cancer des reins, le cancer de la vessie, le cancer des ovaires, un cancer du sang et le cancer du côlon.


### SEQUENCE LISTING

<110> UNIVERSITATSKLINIKUM FREIBURG
<120> Methods and compounds for the diagnosis and treatment of cancer
<130> U7125/DB
<160> 19
<170> PatentIn version 3.3
<210> 1
   <211> 25
   <212> RNA
   <213> Artificial
<220>
   <223> synthetic oligonucleotide; RNA or DNA
<400> 1
   uuuagcaucu cuuacgagga caaug 25
<210> 2
   <211> 21
   <212> RNA
   <213> Artificial
<220>
   <223> synthetic oligonucleotide; RNA or DNA
<400> 2
   uuacguaggg aucugacagg c 21
<210> 3
   <211> 25
   <212> RNA
   <213> Artificial
<220>
   <223> synthetic oligonucleotide
<400> 3
   cauuccuaug cgagacguau guaaa 25
<210> 4
   <211> 25
   <212> RNA
   <213> Artificial
<220>
   <223> synthetic oligonucleotide
<400> 4
   cauuguccuc guaagagaug cuaaa 25
<210> 5
   <211> 64
   <212> DNA
   <213> Artificial
<220>
   <223> synthetic oligonucleotide
<400> 5
   cctgttacgt agggatgaca ggcgtcagtc agtggccaaa acgcctgtca gatccctacg 60 taac 64
<210> 6
   <211> 64
   <212> DNA
   <213> Artificial
<220>
   <223> synthetic oligonucleotide
<400> 6
   tgctgttacg tagggatctg acaggcgttt tggccactga ctgacgcctg tcatccctac 60 gtaa 64
<210> 7
   <211> 136
   <212> PRT
   <213> Homo sapiens
<400> 7
   Met Ala Arg Thr Lys Gln Thr Ala Arg Lys Ser Thr Gly Gly Lys Ala
   1               5                   10                  15
   Pro Arg Lys Gln Leu Ala Thr Lys Ala Ala Arg Lys Ser Ala Pro Ala
               20                  25                  30
   Thr Gly Gly Val Lys Lys Pro His Arg Tyr Arg Pro Gly Thr Val Ala
           35                  40                  45
   Leu Arg Glu Ile Arg Arg Tyr Gln Lys Ser Thr Glu Leu Leu Ile Arg
       50                  55                  60
   Lys Leu Pro Phe Gln Arg Leu Val Arg Glu Ile Ala Gln Asp Phe Lys
   65                  70                  75                  80
   Thr Asp Leu Arg Phe Gln Ser Ser Ala Val Met Ala Leu Gln Glu Ala
                   85                  90                  95
   Cys Glu Ala Tyr Leu Val Gly Leu Phe Glu Asp Thr Asn Leu Cys Ala
               100                 105                 110
   Ile His Ala Lys Arg Val Thr Ile Met Pro Lys Asp Ile Gln Leu Ala
           115                 120                 125
   Arg Arg Ile Arg Gly Glu Arg Ala
       130                 135
<210> 8
   <211> 136
   <212> PRT
   <213> Homo sapiens
<400> 8
<210> 9
   <211> 136
   <212> PRT
   <213> Homo sapiens
<400> 9
   Met Ala Arg Thr Lys Gln Thr Ala Arg Lys Ser Thr Gly Gly Lys Ala
   1               5                   10                  15
   Pro Arg Lys Gln Leu Ala Thr Lys Ala Ala Arg Lys Ser Ala Pro Ser
               20                  25                  30
   Thr Gly Gly Val Lys Lys Pro His Arg Tyr Arg Pro Gly Thr Val Ala
           35                  40                  45
   Leu Arg Glu Ile Arg Arg Tyr Gln Lys Ser Thr Glu Leu Leu Ile Arg
       50                  55                  60
   Lys Leu Pro Phe Gln Arg Leu Val Arg Glu Ile Ala Gln Asp Phe Lys
   65                  70                  75                  80
   Thr Asp Leu Arg Phe Gln Ser Ala Ala Ile Gly Ala Leu Gln Glu Ala
                   85                  90                  95
   Ser Glu Ala Tyr Leu Val Gly Leu Phe Glu Asp Thr Asn Leu Cys Ala
               100                 105                 110
   Ile His Ala Lys Arg Val Thr Ile Met Pro Lys Asp Ile Gln Leu Ala
           115                 120                 125
   Arg Arg Ile Arg Gly Glu Arg Ala
       130                 135
<210> 10
   <211> 671
   <212> PRT
   <213> Homo sapiens
<400> 10
   Met Ala Asp Pro Ala Ala Gly Pro Pro Pro Ser Glu Gly Glu Glu Ser
   1               5                   10                  15
   Thr Val Arg Phe Ala Arg Lys Gly Ala Leu Arg Gln Lys Asn Val His
               20                  25                  30
   Glu Val Lys Asn His Lys Phe Thr Ala Arg Phe Phe Lys Gln Pro Thr
           35                  40                  45
   Phe Cys Ser His Cys Thr Asp Phe Ile Trp Gly Phe Gly Lys Gln Gly
       50                  55                  60
   Phe Gln Cys Gln Val Cys Cys Phe Val Val His Lys Arg Cys His Glu
   65                  70                  75                  80
   Phe Val Thr Phe Ser Cys Pro Gly Ala Asp Lys Gly Pro Ala Ser Asp
                   85                  90                  95
   Asp Pro Arg Ser Lys His Lys Phe Lys Ile His Thr Tyr Ser Ser Pro
               100                 105                 110
   Thr Phe Cys Asp His Cys Gly Ser Leu Leu Tyr Gly Leu Ile His Gln
           115                 120                 125
   Gly Met Lys Cys Asp Thr Cys Met Met Asn Val His Lys Arg Cys Val
       130                 135                 140
   Met Asn Val Pro Ser Leu Cys Gly Thr Asp His Thr Glu Arg Arg Gly
   145                 150                 155                 160
   Arg Ile Tyr Ile Gln Ala His Ile Asp Arg Asp Val Leu Ile Val Leu
                   165                 170                 175
   Val Arg Asp Ala Lys Asn Leu Val Pro Met Asp Pro Asn Gly Leu Ser
               180                 185                 190
   Asp Pro Tyr Val Lys Leu Lys Leu Ile Pro Asp Pro Lys Ser Glu Ser
           195                 200                 205
   Lys Gln Lys Thr Lys Thr Ile Lys Cys Ser Leu Asn Pro Glu Trp Asn
   210 215 220
   Glu Thr Phe Arg Phe Gln Leu Lys Glu Ser Asp Lys Asp Arg Arg Leu
   225 230 235 240
   Ser Val Glu Ile Trp Asp Trp Asp Leu Thr Ser Arg Asn Asp Phe Met
   245 250 255
   Gly Ser Leu Ser Phe Gly Ile Ser Glu Leu Gln Lys Ala Ser Val Asp
   260 265 270
   Gly Trp Phe Lys Leu Leu Ser Gln Glu Glu Gly Glu Tyr Phe Asn Val
   275 280 285
   Pro Val Pro Pro Glu Gly Ser Glu Ala Asn Glu Glu Leu Arg Gln Lys
   290 295 300
   Phe Glu Arg Ala Lys Ile Ser Gln Gly Thr Lys Val Pro Glu Glu Lys
   305 310 315 320
   Thr Thr Asn Thr Val Ser Lys Phe Asp Asn Asn Gly Asn Arg Asp Arg
   325 330 335
   Met Lys Leu Thr Asp Phe Asn Phe Leu Met Val Leu Gly Lys Gly Ser
   340 345 350
   Phe Gly Lys Val Met Leu Ser Glu Arg Lys Gly Thr Asp Glu Leu Tyr
   355 360 365
   Ala Val Lys Ile Leu Lys Lys Asp Val Val Ile Gln Asp Asp Asp Val
   370 375 380
   Glu Cys Thr Met Val Glu Lys Arg Val Leu Ala Leu Pro Gly Lys Pro
   385 390 395 400
   Pro Phe Leu Thr Gln Leu His Ser Cys Phe Gln Thr Met Asp Arg Leu
   405 410 415
   Tyr Phe Val Met Glu Tyr Val Asn Gly Gly Asp Leu Met Tyr His Ile
   420 425 430
   Gln Gln Val Gly Arg Phe Lys Glu Pro His Ala Val Phe Tyr Ala Ala
   435 440 445
   Glu Ile Ala Ile Gly Leu Phe Phe Leu Gln Ser Lys Gly Ile Ile Tyr
   450 455 460
   Arg Asp Leu Lys Leu Asp Asn Val Met Leu Asp Ser Glu Gly His Ile
   465 470 475 480
   Lys Ile Ala Asp Phe Gly Met Cys Lys Glu Asn Ile Trp Asp Gly Val
                   485                 490                 495
   Thr Thr Lys Thr Phe Cys Gly Thr Pro Asp Tyr Ile Ala Pro Glu Ile
               500                 505                 510
   Ile Ala Tyr Gln Pro Tyr Gly Lys Ser Val Asp Trp Trp Ala Phe Gly
           515                 520                 525
   Val Leu Leu Tyr Glu Met Leu Ala Gly Gln Ala Pro Phe Glu Gly Glu
       530                 535                 540
   Asp Glu Asp Glu Leu Phe Gln Ser Ile Met Glu His Asn Val Ala Tyr
   545                 550                 555                 560
   Pro Lys Ser Met Ser Lys Glu Ala Val Ala Ile Cys Lys Gly Leu Met
                   565                 570                 575
   Thr Lys His Pro Gly Lys Arg Leu Gly Cys Gly Pro Glu Gly Glu Arg
               580                 585                 590
   Asp Ile Lys Glu His Ala Phe Phe Arg Tyr Ile Asp Trp Glu Lys Leu
           595                 600                 605
   Glu Arg Lys Glu Ile Gln Pro Pro Tyr Lys Pro Lys Ala Arg Asp Lys
       610                 615                 620
   Arg Asp Thr Ser Asn Phe Asp Lys Glu Phe Thr Arg Gln Pro Val Glu
   625                 630                 635                 640
   Leu Thr Pro Thr Asp Lys Leu Phe Ile Met Asn Leu Asp Gln Asn Glu
                   645                 650                 655
   Phe Ala Gly Phe Ser Tyr Thr Asn Pro Glu Phe Val Ile Asn Val
               660                 665                 670
<210> 11
   <211> 2711
   <212> DNA
   <213> Homo sapiens
<400> 11
   agctggacga gcggcagcag ctgggcgagt gacagccccg gctccgcgcg ccgcggccgc     60
   cagagccggc gcaggggaag cgcccgcggc cccgggtgca gcagcggccg ccgcctcccg    120
   cgcctccccg gcccgcagcc cgcggtcccg cggccccggg gccggcacct ctcgggctcc    180
   ggctccccgc gcgcaagatg gctgacccgg ctgcggggcc gccgccgagc gagggcgagg    240
   agagcaccgt gcgcttcgcc cgcaaaggcg ccctccggca gaagaacgtg catgaggtca    300
   agaaccacaa attcaccgcc cgcttcttca agcagcccac cttctgcagc cactgcaccg    360
   acttcatctg gggcttcggg aagcagggat tccagtgcca agtttgctgc tttgtggtgc     420
   acaagcggtg ccatgaattt gtcacattct cctgccctgg cgctgacaag ggtccagcct    480
   ccgatgaccc ccgcagcaaa cacaagttta agatccacac gtactccagc cccacgtttt 540
   gtgaccactg tgggtcactg ctgtatggac tcatccacca ggggatgaaa tgtgacacct 600
   gcatgatgaa tgtgcacaag cgctgcgtga tgaatgttcc cagcctgtgt ggcacggacc 660
   acacggagcg ccgcggccgc atctacatcc aggcccacat cgacagggac gtcctcattg 720
   tcctcgtaag agatgctaaa aaccttgtac ctatggaccc caatggcctg tcagatccct 780
   acgtaaaact gaaactgatt cccgatccca aaagtgagag caaacagaag accaaaacca 840
   tcaaatgctc cctcaaccct gagtggaatg agacatttag atttcagctg aaagaatcgg 900
   acaaagacag aagactgtca gtagagattt gggattggga tttgaccagc aggaatgact 960
   tcatgggatc tttgtccttt gggatttctg aacttcagaa agccagtgtt gatggctggt 1020
   ttaagttact gagccaggag gaaggcgagt acttcaatgt gcctgtgcca ccagaaggaa 1080
   gtgaggccaa tgaagaactg cggcagaaat ttgagagggc caagatcagt cagggaacca 1140
   aggtcccgga agaaaagacg accaacactg tctccaaatt tgacaacaat ggcaacagag 1200
   accggatgaa actgaccgat tttaacttcc taatggtgct ggggaaaggc agctttggca 1260
   aggtcatgct ttcagaacga aaaggcacag atgagctcta tgctgtgaag atcctgaaga 1320
   aggacgttgt gatccaagat gatgacgtgg agtgcactat ggtggagaag cgggtgttgg 1380
   ccctgcctgg gaagccgccc ttcctgaccc agctccactc ctgcttccag accatggacc 1440
   gcctgtactt tgtgatggag tacgtgaatg gggcgacctc catgtatcac atccagcaag 1500
   tcggccggtt caaggagccc catgctgtat tttacgctgc agaaattgcc atcggtctgt 1560
   tcttcttaca gagtaagggc atcatttacc gtgacctaaa acttgacaac gtgatgctcg 1620
   attctgaggg acacatcaag attgccgatt ttggcatgtg taaggaaaac atctgggatg 1680
   gggtgacaac caagacattc tgtggcactc cagactacat cgcccccgag ataattgctt 1740
   atcagcccta tgggaagtcc gtggattggt gggcatttgg agtcctgctg tatgaaatgt 1800
   tggctgggca ggcacccttt gaaggggagg atgaagatga actcttccaa tccatcatgg 1860
   aacacaacgt agcctatccc aagtctatgt ccaaggaagc tgtggccatc tgcaaagggc 1920
   tgatgaccaa acacccaggc aaacgtctgg gttgtggacc tgaaggcgaa cgtgatatca 1980
   aagagcatgc atttttccgg tatattgatt gggagaaact tgaacgcaaa gagatccagc 2040
   ccccttataa gccaaaagct agagacaaga gagacacctc caacttcgac aaagagttca 2100
   ccagacagcc tgtggaactg acccccactg ataaactctt catcatgaac ttggaccaaa 2160
   atgaatttgc tggcttctct tatactaacc cagagtttgt cattaatgtg taggtgaatg 2220
   caaactccat cgttgagcct ggggtgtaag acttcaagcc aagcgtatgt atcaattcta 2280
   gtcttccagg attcacggtg cacatgctgg cattcaacat gtggaaagct tgtcttagag 2340
   ggcttttctt tgtatgtgta gcttgctagt ttgttttcta catttgaaaa tgtttagttt 2400
   agaataagcg cattatccaa ttatagaggt acaattttcc aaacttccag aaactcatca 2460
   aatgaacaga caatgtcaaa actactgtgt ctgataccaa aatgcttcag tatttgtaat 2520
   ttttcaagtc agaagctgat gttcctggta aaagttttta cagttattct ataatatctt 2580
   ctttgaatgc taagcatgag cgatattttt aaaaattgtg agtaagcttt gcagttactg   2640
   tgaactattg tctcttggag gaagtttttt gtttaagaat tgatatgatt aaactgaatt   2700
   aatatatgca a                                                        2711
<210> 12
   <211> 673
   <212> PRT
   <213> Homo sapiens
<400> 12
   Met Ala Asp Pro Ala Ala Gly Pro Pro Pro Ser Glu Gly Glu Glu Ser
   1               5                   10                  15
   Thr Val Arg Phe Ala Arg Lys Gly Ala Leu Arg Gln Lys Asn Val His
               20                  25                  30
   Glu Val Lys Asn His Lys Phe Thr Ala Arg Phe Phe Lys Gln Pro Thr
           35                  40                  45
   Phe Cys Ser His Cys Thr Asp Phe Ile Trp Gly Phe Gly Lys Gln Gly
       50                  55                  60
   Phe Gln Cys Gln Val Cys Cys Phe Val Val His Lys Arg Cys His Glu
   65                  70                  75                  80
   Phe Val Thr Phe Ser Cys Pro Gly Ala Asp Lys Gly Pro Ala Ser Asp
                   85                  90                  95
   Asp Pro Arg Ser Lys His Lys Phe Lys Ile His Thr Tyr Ser Ser Pro
               100                 105                 110
   Thr Phe Cys Asp His Cys Gly Ser Leu Leu Tyr Gly Leu Ile His Gln
           115                 120                 125
   Gly Met Lys Cys Asp Thr Cys Met Met Asn Val His Lys Arg Cys Val
       130                 135                 140
   Met Asn Val Pro Ser Leu Cys Gly Thr Asp His Thr Glu Arg Arg Gly
   145                 150                 155                 160
   Arg Ile Tyr Ile Gln Ala His Ile Asp Arg Asp Val Leu Ile Val Leu
                   165                 170                 175
   Val Arg Asp Ala Lys Asn Leu Val Pro Met Asp Pro Asn Gly Leu Ser
               180                 185                 190
   Asp Pro Tyr Val Lys Leu Lys Leu Ile Pro Asp Pro Lys Ser Glu Ser
           195                 200                 205
   Lys Gln Lys Thr Lys Thr Ile Lys Cys Ser Leu Asn Pro Glu Trp Asn
       210                 215                 220
   Glu Thr Phe Arg Phe Gln Leu Lys Glu Ser Asp Lys Asp Arg Arg Leu
   225                 230                 235                 240
   Ser Val Glu Ile Trp Asp Trp Asp Leu Thr Ser Arg Asn Asp Phe Met
                   245                 250                 255
   Gly Ser Leu Ser Phe Gly Ile Ser Glu Leu Gln Lys Ala Ser Val Asp
               260                 265                 270
   Gly Trp Phe Lys Leu Leu Ser Gln Glu Glu Gly Glu Tyr Phe Asn Val
           275                 280                 285
   Pro Val Pro Pro Glu Gly Ser Glu Ala Asn Glu Glu Leu Arg Gln Lys
       290                 295                 300
   Phe Glu Arg Ala Lys Ile Ser Gln Gly Thr Lys Val Pro Glu Glu Lys
   305                 310                 315                 320
   Thr Thr Asn Thr Val Ser Lys Phe Asp Asn Asn Gly Asn Arg Asp Arg
                   325                 330                 335
   Met Lys Leu Thr Asp Phe Asn Phe Leu Met Val Leu Gly Lys Gly Ser
               340                 345                 350
   Phe Gly Lys Val Met Leu Ser Glu Arg Lys Gly Thr Asp Glu Leu Tyr
           355                 360                 365
   Ala Val Lys Ile Leu Lys Lys Asp Val Val Ile Gln Asp Asp Asp Val
       370                 375                 380
   Glu Cys Thr Met Val Glu Lys Arg Val Leu Ala Leu Pro Gly Lys Pro
   385                 390                 395                 400
   Pro Phe Leu Thr Gln Leu His Ser Cys Phe Gln Thr Met Asp Arg Leu
                   405                 410                 415
   Tyr Phe Val Met Glu Tyr Val Asn Gly Gly Asp Leu Met Tyr His Ile
               420                 425                 430
   Gln Gln Val Gly Arg Phe Lys Glu Pro His Ala Val Phe Tyr Ala Ala
           435                 440                 445
   Glu Ile Ala Ile Gly Leu Phe Phe Leu Gln Ser Lys Gly Ile Ile Tyr
       450                 455                 460
   Arg Asp Leu Lys Leu Asp Asn Val Met Leu Asp Ser Glu Gly His Ile
   465                 470                 475                 480
   Lys Ile Ala Asp Phe Gly Met Cys Lys Glu Asn Ile Trp Asp Gly Val
                   485                 490                 495
   Thr Thr Lys Thr Phe Cys Gly Thr Pro Asp Tyr Ile Ala Pro Glu Ile
   500 505 510
   Ile Ala Tyr Gln Pro Tyr Gly Lys Ser Val Asp Trp Trp Ala Phe Gly
   515 520 525
   Val Leu Leu Tyr Glu Met Leu Ala Gly Gln Ala Pro Phe Glu Gly Glu
   530 535 540
   Asp Glu Asp Glu Leu Phe Gln Ser Ile Met Glu His Asn Val Ala Tyr
   545 550 555 560
   Pro Lys Ser Met Ser Lys Glu Ala Val Ala Ile Cys Lys Gly Leu Met
   565 570 575
   Thr Lys His Pro Gly Lys Arg Leu Gly Cys Gly Pro Glu Gly Glu Arg
   580 585 590
   Asp Ile Lys Glu His Ala Phe Phe Arg Tyr Ile Asp Trp Glu Lys Leu
   595 600 605
   Glu Arg Lys Glu Ile Gln Pro Pro Tyr Lys Pro Lys Ala Cys Gly Arg
   610 615 620
   Asn Ala Glu Asn Phe Asp Arg Phe Phe Thr Arg His Pro Pro Val Leu
   625 630 635 640
   Thr Pro Pro Asp Gln Glu Val Ile Arg Asn Ile Asp Gln Ser Glu Phe
   645 650 655
   Glu Gly Phe Ser Phe Val Asn Ser Glu Phe Leu Lys Pro Glu Val Lys
   660 665 670
   Ser
<210> 13
   <211> 8014
   <212> DNA
   <213> Homo sapiens
<400> 13
   agctggacga gcggcagcag ctgggcgagt gacagccccg gctccgcgcg ccgcggccgc 60
   cagagccggc gcaggggaag cgcccgcggc cccgggtgca gcagcggccg ccgcctcccg 120
   cgcctccccg gcccgcagcc cgcggtcccg cggccccggg gccggcacct ctcgggctcc 180
   ggctccccgc gcgcaagatg gctgacccgg ctgcggggcc gccgccgagc gagggcgagg 240
   agagcaccgt gcgcttcgcc cgcaaaggcg ccctccggca gaagaacgtg catgaggtca 300
   agaaccacaa attcaccgcc cgcttcttca agcagcccac cttctgcagc cactgcaccg 360
   acttcatctg gggcttcggg aagcagggat tccagtgcca agtttgctgc tttgtggtgc 420
   acaagcggtg ccatgaattt gtcacattct cctgccctgg cgctgacaag ggtccagcct     480
   ccgatgaccc ccgcagcaaa cacaagttta agatccacac gtactccagc cccacgtttt     540
   gtgaccactg tgggtcactg ctgtatggac tcatccacca ggggatgaaa tgtgacacct     600
   gcatgatgaa tgtgcacaag cgctgcgtga tgaatgttcc cagcctgtgt ggcacggacc     660
   acacggagcg ccgcggccgc atctacatcc aggcccacat cgacagggac gtcctcattg     720
   tcctcgtaag agatgctaaa aaccttgtac ctatggaccc caatggcctg tcagatccct     780
   acgtaaaact gaaactgatt cccgatccca aaagtgagag caaacagaag accaaaacca     840
   tcaaatgctc cctcaaccct gagtggaatg agacatttag atttcagctg aaagaatcgg     900
   acaaagacag aagactgtca gtagagattt gggattggga tttgaccagc aggaatgact     960
   tcatgggatc tttgtccttt gggatttctg aacttcagaa agccagtgtt gatggctggt    1020
   ttaagttact gagccaggag gaaggcgagt acttcaatgt gcctgtgcca ccagaaggaa    1080
   gtgaggccaa tgaagaactg cggcagaaat ttgagagggc caagatcagt cagggaacca    1140
   aggtcccgga agaaaagacg accaacactg tctccaaatt tgacaacaat ggcaacagag    1200
   accggatgaa actgaccgat tttaacttcc taatggtgct ggggaaaggc agctttggca    1260
   aggtcatgct ttcagaacga aaaggcacag atgagctcta tgctgtgaag atcctgaaga    1320
   aggacgttgt gatccaagat gatgacgtgg agtgcactat ggtggagaag cgggtgttgg    1380
   ccctgcctgg gaagccgccc ttcctgaccc agctccactc ctgcttccag accatggacc    1440
   gcctgtactt tgtgatggag tacgtgaatg ggggcgacct catgtatcac atccagcaag    1500
   tcggccggtt caaggagccc catgctgtat tttacgctgc agaaattgcc atcggtctgt    1560
   tcttcttaca gagtaagggc atcatttacc gtgacctaaa acttgacaac gtgatgctcg    1620
   attctgaggg acacatcaag attgccgatt ttggcatgtg taaggaaaac atctgggatg    1680
   gggtgacaac caagacattc tgtggcactc cagactacat cgcccccgag ataattgctt    1740
   atcagcccta tgggaagtcc gtggattggt gggcatttgg agtcctgctg tatgaaatgt    1800
   tggctgggca ggcacccttt gaaggggagg atgaagatga actcttccaa tccatcatgg    1860
   aacacaacgt agcctatccc aagtctatgt ccaaggaagc tgtggccatc tgcaaagggc    1920
   tgatgaccaa acacccaggc aaacgtctgg gttgtggacc tgaaggcgaa cgtgatatca    1980
   aagagcatgc atttttccgg tatattgatt gggagaaact tgaacgcaaa gagatccagc    2040
   ccccttataa gccaaaagct tgtgggcgaa atgctgaaaa cttcgaccga tttttcaccc    2100
   gccatccacc agtcctaaca cctcccgacc aggaagtcat caggaatatt gaccaatcag    2160
   aattcgaagg attttccttt gttaactctg aatttttaaa acccgaagtc aagagctaag    2220
   tagatgtgta gatctccgtc cttcatttct gtcattcaag ctcaacggct attgtggtga    2280
   catttttatg tttttcattg ccaagttgca tccatgtttg attttctgat gagactagag    2340
   tgacagtgtt tcagaaccca aatgtcctca ggtagtttgg agcatctcta tgagatggga    2400
   ttatgcagat ggcctatgga aaatgcagct gcataattaa cacattatca aagtcctctt    2460
   acaatttatt ttccgcagca tgtcagctaa gtagacccaa tggggagaga aaatgcctgc    2520
   tttctttccc tctttttctg cactgccata ttcaccccca accatccaat ctgtggataa 2580
   ttggatgtta gcggtactct tccacttccg ggcctggagc ttggcttgta tccaagtgta 2640
   tggttgcttt gcctaagagg aatccctcta tttcacctgt tctggaggca ccagaccttg 2700
   aaaagaacat gctcaaaata aaatgttatc tgttattttt gtaaactcaa agttaagatg 2760
   atcaaagttc taaaattcca agaatgtgct tttagacggt ctcaatctaa aagcacttca 2820
   aggggtcaaa gggcaaccag cttgggtgct acctcagtgt tgtagtttct gatactttat 2880
   gtctttgctc accctcatcc ccaaactact tgaaaagggc atttggcacc actctctgaa 2940
   acaacacagt cactctagca aggcccccaa agggccctgg ttttacatta catttcaaac 3000
   tttatttgct ttggggtttt gtttctgttg ttgttcaaat gcaaaaaaaa gaaaaaaaaa 3060
   gaaaaaaaaa ggtgactcac attgttacac atgctttaaa atatgtattc aaatgttatt 3120
   aaccacaatg acgacctgct ttgatttaac caagaagacg gctgcggagc ctagcagact 3180
   caggcctgtg ggaatgggat ttgttacaaa tctaggtttg ttactggctt cagaaagcta 3240
   attaagtgct ctgaaaaaga caccgtttct tgaaacaaag atggttgtat tcctcacttt 3300
   gatgttgttt tgcaagatgt ttgtggaaat gttcatttgt atctggatct ctgttatgtg 3360
   ccatttttct tctagcatcg agatacaata aaaaaaaaaa aaaagaaaag aagaagaaat 3420
   actatttcaa ggaaaactgc tctttttgag aaacgtggac ctaaactaca aagtgggaac 3480
   tgaggaggga actcaggaga aaggaactaa ctgcggagct ttaatcttgg ccccagtgtt 3540
   cagccactcg gaggggcggg ggctgtggcc cattcagggg ctgctggtgg gctgtagtgg 3600
   ggtgggatga cctggccaga gccaacgagg atactggagc ccaaagtcaa gtttagagac 3660
   cagctgggaa cgtgaatggg gctcttgatt ttcttatcaa aatcaccact cctcccagct 3720
   tggactaaat attctttcta gcaagcagct ttgtgagctc cctgaagccc aaggaaaccc 3780
   ttcggtggga gaaatttcat ttctgtctga gaggattaag gcagcaggtg actccccctc 3840
   ctcgcctgcc gtgtcctgct attctcaggc agctctaagg agaattctta tcacagttca 3900
   agtgatttcc agaagttcca gggcttctga gagaccatca agggaacttt aacaacttga 3960
   caaatgtcct tgaagtaaga tgcctcatct ttagggaaaa atggggtttg gatttctgct 4020
   taggcaaagt ctcctgcagt tcatccttct ctgtcctctt cttgcttcag gcttggggac 4080
   cgtccctgct gtccccactg tggtggcaat caggacctaa ggtgaagcaa acttgaagtt 4140
   ctatctgaca agtttaggca gtaagagaag gagggaaatc ggagcaaagc tccctcactt 4200
   tattgttgag aaactggcat ctggaaagag gaaggaattt gcccaaagtc agtcagctgg 4260
   gataaaaacc tgggtgtcct gtccagaaag tgcagggtgc tttctgctct gtagcaaggc 4320
   agcagacatc tctgagccag gcccaccaac aggcccttat ctggtggttg gatcatgatc 4380
   ccattttgct tggacatgct ctcaggaaga taaaaaccat ggagaaacac taggccattg 4440
   acaaatgatc tgagacaact ttagaaaaca atgtaggatg aatggaaaga gaaagaaagg 4500
   aaagaaagaa gaaaaagaaa gaaggaaaga aagaaagaga aaggaaggaa ggaaagaagg 4560
   aaggaaaaga aggaaggaag gaaggaatat agtgttataa atactgcact caacattttc    4620
   caaattcttg ccattatttt tcaaaagttt aatagtttgc agaaatagat actcaagcca    4680
   aagtctgttt tagagaaact ttccatggaa agtcagaatt tctaccactt ccttttctat    4740
   ccacatttcc agtgcagaag aaactgagaa acagagcttt ttgaagagag gacagggcca    4800
   tagcaacaag gaccttcttg ggggattaat gggaggtcag tagaattaat aaccctcctt    4860
   ggatgagtgc tactgttttc acatggcttc agatgctatc aacctcaaag aaatgatctc    4920
   aacagagaag cttattctct cccaacttct acggtaaaat ccaggagtat tttctctggg    4980
   gatctgccca caggacaaag tccataaaag caagtcctgt ctggaccatg tggttatctg    5040
   aagcattagc catcaccagc acaacaaacg gggcagggct ttccaaggtg gggctggtca    5100
   gaagggaatc tttgataaga ggcccacagg cagggaaagc gaaatagggt tgatgagacc    5160
   aggggagacc taaaaaaaag gcagctttgt gtcttctagc tccaaatata cctgcctttt    5220
   agctcacaca ctgtcctgga gttctcagac ctttaggggc cctaacacag ttcagttcat    5280
   acaggggttc aaaagggaca gtggcccatt tgggagacct ttaggatcaa tgggaatcaa    5340
   ttccattgtt ttgcctcaga gtaaagtttc tggctcgggg acaattataa gttgcaaaaa    5400
   ggatagaggc atatcccaag tcttccttca ttccacaaat aattacaaac aacctactgt    5460
   gtgccaggca ctattcttag cactggaaat acactagtga agaagcagat gaggaccctg    5520
   tttattgttt ctctccaaga aattctccaa gaatattgtt tcttggagag aaataataaa    5580
   taaacaagac aatttctgaa agcaataagt gcaatcaaga taattaaagg atgctaaagt    5640
   gtgacttgtg gggattggga gagagatgca cagacaatat taaagaggag gcattcgagc    5700
   tttgttgtga acaccggaag taacatgccg agcgcctggg ggatggaaac tcctatagca    5760
   ccccacaggc taacagcaag caggacaaga caaaaagggc aggtgggaca tggtagagat    5820
   ggaccctacc caggaaacag ctccatcagc atcttagcct gccccactct agccacacat    5880
   acccacgtgt gctcctgagt tcagtgtgcc cacctcactc ccacaccctc acatagactt    5940
   ggcaagagta aggagggaac tccatagaga cattttacct atctcagggg agcagccaca    6000
   aagaagcaag tcttgtaaaa ggtcttttgc aaaggagagt gaacccagca atgagagatc    6060
   cttaacagct agtgcccatt agggggctaa acctaaagcc tgggtggtga tggctcaaac    6120
   gctaatgagt cagtgaatcc ttaccgaccc cctggccttt ataatctgag gcaactttgg    6180
   ctgcagcccg ggaatgtgca gggcactagg gaatacaagg ccttcttccc tggttgtctt    6240
   gtaataaaac agccatgggg ttgtccctcc agtccgagag actgtgatga ggcctacata    6300
   gcagcgatgt ggtcaggtaa aaatcaggaa cccactgaaa tcttgggcaa gccaccctgc    6360
   ctgcttgtgc ctcggttctc tcatatgtca tatataggag gtgaggactc cagctccacc    6420
   tgccccaggt gggtgtggtg atgatgagga aagacaagag gcttgcaagg accctgaaga    6480
   ggtcggagca tcatacagat tcctttatta gcccacattc tgatgttccc tggtgagact    6540
   tgccccaagc aattgctagt aaatgggggt taatttcttc tccacctccc tactgaacaa    6600
   aaaaagaaat gccagactta ctaggagaat cgagttgctt tgagtttctt ttgttttgtt    6660
   ttgttttgtt ttgttttaag gctcccctta cacaccctcc tttaagcttt gggttttctc 6720
   tcttatagtt tgttgacaca tgctaaaaat gtctttggag agaacttctg cctgataaac 6780
   acccaattct agactgtggg tggattttcg agctgacggt ggtcaattcc tttcattaag 6840
   cagtgatctg atttctccac atggccattc tgccttcttg ggggcagagt agatgggcag 6900
   cagttcacct tttcagagaa agaggtcttc tagccacctg ggctgctact gaatggtttt 6960
   ctccaggacg ctctacctaa tgattatttc tataacatta agcatggtaa taagtagctt 7020
   ccaattcaat tcatcctaaa gccaaagaaa atacagcaac acacacacac acacacacac 7080
   acacacacac acacacacac acacaccact ttatggcaat tcttaactga cattcaatga 7140
   cttacttctt ttcttagaaa atttccacca catttctatc cccaagccaa catacaatgt 7200
   gaaatgaaag ccagtgcgtg gagtgcagct gctaaaaatt ttcagcacag ggctctttct 7260
   gactctgctc atgagatggt atcagccacc caatgactgg cgtatcttgg tcctgtgtct 7320
   ttcttcttac gctgtgttaa tgtgtttact ttccatttgg cagagagaca agagagacac 7380
   ctccaacttc gacaaagagt tcaccagaca gcctgtggaa ctgaccccca ctgataaact 7440
   cttcatcatg aacttggacc aaaatgaatt tgctggcttc tcttatacta acccagagtt 7500
   tgtcattaat gtgtaggtga atgcaaactc catcgttgag cctggggtgt aagacttcaa 7560
   gccaagcgta tgtatcaatt ctagtcttcc aggattcacg gtgcacatgc tggcattcaa 7620
   catgtggaaa gcttgtctta gagggctttt ctttgtatgt gtagcttgct agtttgtttt 7680
   ctacatttga aaatgtttag tttagaataa gcgcattatc caattataga ggtacaattt 7740
   tccaaacttc cagaaactca tcaaatgaac agacaatgtc aaaactactg tgtctgatac 7800
   caaaatgctt cagtatttgt aatttttcaa gtcagaagct gatgttcctg gtaaaagttt 7860
   ttacagttat tctataatat cttctttgaa tgctaagcat gagcgatatt tttaaaaatt 7920
   gtgagtaagc tttgcagtta ctgtgaacta ttgtctcttg gaggaagttt tttgtttaag 7980
   aattgatatg attaaactga attaatatat gcaa 8014
<210> 14
   <211> 25
   <212> RNA
   <213> Artificial
<220>
   <223> synthetic oligonucleotide
<400> 14
   gaaaguccua gauccacacg caaau 25
<210> 15
   <211> 25
   <212> RNA
   <213> Artificial
<220>
   <223> synthetic oligonucleotide
<400> 15
   gaacaugauc cagaccuaca gcaau 25
<210> 16
   <211> 25
   <212> DNA
   <213> Artificial
<220>
   <223> synthetic oligonucleotide
<400> 16
   tttagcatct cttacgagga caatg 25
<210> 17
   <211> 19
   <212> DNA
   <213> Artificial
<220>
   <223> synthetic oligonucleotide
<400> 17
   gcctgtcatc cctacgtaa 19
<210> 18
   <211> 21
   <212> DNA
   <213> Artificial
<220>
   <223> synthetic oligonucleotide
<400> 18
   ttacgtaggg atctgacagg c 21
<210> 19
   <211> 25
   <212> DNA
   <213> Artificial
<220> .
   <223> synthetic oligonucleotide
<400> 19
   cattgtcctc gtaagagatg ctaaa 25